# FASCICULE DE BREVET EUROPEEN

(11) **EP 0 524 864 B1**
(45) Date de publication et mention de la délivrance du brevet: **27.05.1998**
(21) Numéro de dépôt: 92402080.3
(22) Date de dépôt: 17.07.1992
(51) Int. Cl.: C12Q 1/68, C07H 21/00

(54) **Procédé d'immobilisation d'un fragment d'acide nucléique par fixation passive sur un support solide, support solide ainsi obtenu et son utilisation**
Verfahren zur Immobilisierung eines Nukleinsäurefragments durch passive Fixierung auf einen Festträger, daraus erhaltener Festträger und seine Verwendung
Method for immobilizing a nucleic acid fragment by passive adsorption on a solid support, solid support obtained therefrom and its utilisation

(30) Priorité: 17.07.1991 FR 9109057
(43) Date de publication de la demande: 27.01.1993
(73) Titulaire: BIO MERIEUX, Société anonyme, F-69280 Marcy l'Etoile (FR)
(72) Inventeur: Cros, Philippe, F-69005 Lyon (FR); Allibert, Patrice André, F-69290 Grezieu La Varenne (FR); Mandrand, Bernard Fabien, F-69100 Villeurbanne (FR); Dalbon, Pascal Thierry, F-69200 Venissieux (FR)
(74) Mandataire: Tonnellier, Jean-Claude

(56) Documents cités:
- EP-A- 0 221 308
- EP-A- 0 435 150
- WO-A-89/03849
- WO-A-91/00288
- WO-A-91/08307
- WO-A-91/19729
- WO-A-91/19812
- US-A- 4 379 843
- JOURNAL OF IMMUNOLOGICAL METHODS. vol. 90, 1986, NEW YORK US pages 105 - 110 M. ZOUALI ET AL. 'A rapid ELISA for measurement of antibodies to nucleic acid antigens using UV-treated polystyrene plates'
- JOURNAL OF IMMUNOLOGICAL METHODS. vol. 63, no. 3, 1983, NEW YORK US pages 359 - 366 RUBIN R.L. ET AL. 'An improved ELISA for anti-native DNA by elimination of interference by anti-histone antibodies'

## Description

La présente invention a pour objet un procédé d'immobilisation d'un fragment d'acide nucléique sur un support solide, le support solide ainsi obtenu et son utilisation notamment dans les méthodes de détection de séquences cibles comportant une séquence complémentaire de la séquence immobilisée.

On sait que l'une des propriétés des acides nucléiques est la possibilité d'interagir avec une séquence complémentaire par l'intermédiaire de liaisons hydrogènes et de former un hybride selon les lois d'appariement connues, c'est-à-dire A-T, G-C pour l'ADN et A-U, G-C pour l'ARN.

Sur la base des propriétés des acides nucléiques, des techniques ont été développées permettant de mettre en évidence et de quantifier, dans un échantillon à analyser, un acide nucléique appelé cible. Ces techniques peuvent être divisées en deux grands groupes : les techniques dites de détection directe telles que celles développées par SOUTHERN. E. M. (J. Mol. Biol., 98, 503, (1975)) et la technique dite "Dot-blot" (MANIATIS et al., Molecular Cloning, Cold Spring Harbor, (1982)) pour la détection d'ADN, selon lesquelles l'ADN cible est déposé sur un film de nitrocellulose, de nylon ou un mélange de nitrocellulose et de nylon et on utilise pour la détection une séquence nucléique déterminée, appelée sonde de détection, marquée par tout marqueur connu tel que marqueur enzymatique, chimique ou radioactif. Ainsi, lorsqu'il y a complémentarité entre la séquence nucléique de la cible et la séquence nucléique de la sonde de détection, il se forme un hybride stable et après une étape de lavage pour éliminer la sonde de détection non appariée, on peut quantifier la cible dans l'échantillon. Une autre technique de détection directe est la technique dite "NORTHERN" identique dans la pratique à la technique dite "SOUTHERN" mais utilisée pour mettre en évidence et quantifier l'acide ribonucléique. Une modification à ces techniques sont les techniques dites indirectes telles que la technique sandwich ou "Reverse Dot". Selon cette technique, on utilise une première sonde nucléotidique, appelée sonde de capture, fixée sur un support solide qui sert à capter le gène ou fragment de gène à détecter dans l'échantillon. La cible peut avoir été marquée au préalable (le plus souvent par un haptène ou par une modification chimique). Dans ce cas on effectuera une détection appropriée. Si la cible n'est pas marquée, on peut alors ajouter une deuxième sonde dite sonde de détection, complémentaire d'une autre région de la cible, qui permet la détection par l'intermédiaire d'un marqueur tel qu'un marqueur radioactif, enzymatique ou chimique (voir par exemple DUNN A.R. et HASSEL J.A, Cell, 12,23 (1977); RANKI M. et al., Gene, 21,77 (1983) ; PALVA A. et al., FEMS Microbiol. Lett. 23,83 (1984) ; POLSKY-CYNKI R. et al., Clin. Chem., 31,1438 (1985)).

Toutes ces techniques nécessitent l'immobilisation d'un fragment d'acide nucléique sur un support solide. La méthode la plus communément utilisée pour lier un fragment d'acide nucléique à un support est de l'attacher par diverses interactions non covalentes. Les désavantages de cette méthode résident dans le fait qu'une partie de l'acide nucléique est immobilisée directement sur le support et que seule une petite partie du fragment d'acide nucléique reste disponible pour l'hybridation. Ces désavantages sont amplifiés lorsqu'on utilise des fragments d'acide nucléique relativement courts, par exemple inférieurs à 100 nucléotides. Ce problème se pose notamment lorsque l'on veut détecter des mutations génétiques et des polymorphismes. Pour cela, on utilise des sondes nucléiques de synthèse comprenant environ 15 à 20 nucléotides qui s'hybrideront aux cibles à détecter seulement s'il y a une complémentarité parfaite. Dans la plupart des cas, le mésappariement d'une simple paire de base est suffisant pour empêcher la formation d'un complexe sonde-cible stable, dans des conditions préalablement choisies.

Des solutions ont été proposées pour pallier ces inconvénients, parmi lesquelles celle notamment préconisée par SAIKI R.K. et al., Proc.Natl.Acad.Sci.USA, vol. 86, pp. 6230-6234, août 1989, qui consiste à coupler à l'extrémité 3' d'un oligonucléotide ou sonde une queue poly(dT) de 400 bases et à immobiliser l'oligonucléotide par l'intermédiaire de cette queue poly(dT) sur un filtre de nylon par exposition à des rayons ultraviolets, de façon à coupler par covalence les bases thymines aux amines primaires présentes dans le nylon.

Cependant, cette solution n'est pas entièrement satisfaisante parce qu'elle présente des problèmes de spécificité. En effet, les bases thymines de l'oligonucléotide peuvent également réagir, sous rayonnement U.V., avec le support, ce qui implique une diminution de l'efficacité d'hybridation. Ce problème est partiellement résolu, comme décrit dans la publication, par l'utilisation d'une queue très longue (400 bases) mais alors, la sonde, en masse , devient très inférieure à la queue, ce qui diminue d'autant le taux de greffage en moles/g de papier. De plus, la fabrication d'une sonde avec une queue d'aussi grande taille pose des problèmes d'industrialisation.

Une autre solution préconisée est celle décrite dans la demande de brevet WO 88/01302 qui consiste à coupler à l'extrémité terminale d'un oligonucléotide un ligand, tel qu'un aminoalkyle, et à attacher l'extrémité libre de ce ligand sur un support solide, tel que polystyrène ou analogues, par formation d'une liaison covalente. Bien que cette méthode permette d'assurer une fixation stable de l'oligonucléotide, elle n'en demeure pas moins difficile à mettre en oeuvre techniquement car elle nécessite une étape d'activation du support pour créer à la surface de celui-ci une fonction réactive déterminée capable de former une liaison covalente avec la fonction réactive portée à l'extrémité du ligand.

On a maintenant trouvé un nouveau procédé qui pallie les inconvénients ci-dessus mentionnés, de mise en oeuvre simple et qui assure une excellente reproductibilité.

La présente invention a pour objet un procédé d'immobilisation par fixation passive non spécifique, sur un support solide, d'un fragment d'acide nucléique comportant moins de 100 nucléotides (notamment moins de 50 nucléotides et en particulier moins de 30 nucléotides), dans lequel on dépose sur ledit support ledit fragment sous la forme d'un dérivé résultant du couplage covalent dudit fragment avec un ligand ayant une masse moléculaire inférieure à 5000 et comprenant au moins un groupement fonctionnel polaire, ledit dérivé n'étant pas susceptible de réagir sur ledit support avec formation d'une liaison covalente dans les conditions dudit dépôt, étant entendu que lorsque ledit ligand est un nucléotide ou oligonucléotide, il comprend au moins un nucléotide qui a été modifié de façon à porter ledit groupement fonctionnel polaire.

On entend par "fixation de façon passive" une fixation due à des forces autres que des forces de liaison covalente.

Dans des modes de réalisation particuliers, le procédé de l'invention peut encore présenter les caractéristiques suivantes, prises isolément ou, le cas échéant, en combinaison :
- ledit groupement fonctionnel est choisi parmi les groupements amine, hydrazino, hydrazono, imino, azido, triazeno, triazano, amide éventuellement substitué, semicarbazono, carbamoyle, cyano, carboxy, hydroxy, alkylthio , arylthio , sulfamoyle, thiophosphate ;
- ledit groupement fonctionnel polaire est choisi parmi les groupements amine (primaire,secondaire ou tertiaire), hydroxy, alkylthio et arylthio ;
- ledit groupement amine est choisi parmi les groupements amino,amino mono ou disubstitué(le ou les substituants étant choisis parmi les groupements alkyle ou aryle éventuellement substitués), amidino, guanidino, triazinyle, indolyle et imidazolyle ;
- ledit ligand comprend au moins un groupement hydrophobe ; ledit groupement hydrophobe est choisi notamment parmi les groupements hydrocarbonés éventuellement insaturés ayant de 2 à 30 atomes de carbone ; le ou les groupements hydrocarbonés peuvent être choisis notamment parmi les groupements alkyle, alkylène, alcényle et alcénylène, les groupements aryle et arylène et les groupements alkyle, alkylène, alcényle ou alcénylène éventuellement interrompus ou substitués par au moins un groupement arylène ou aryle ; le ou les groupements hydrophobes peuvent être substitués par ledit ou lesdits groupements fonctionnels polaires ;
- le ligand est de préférence lié à un nucléotide terminal de la séquence immobilisée, en particulier à l'extrémité 3' et 5' de ladite séquence, ou encore lié à la base (thymine, cytosine, etc...) dudit nucléotide terminal ; le ligand peur également être lié à une base d'un nucléotide non terminal , ou au phosphore d'une liaison intranucléotidique ;
- ledit ligand comprend un groupement choisi parmi :
- les groupements de formule (I) : 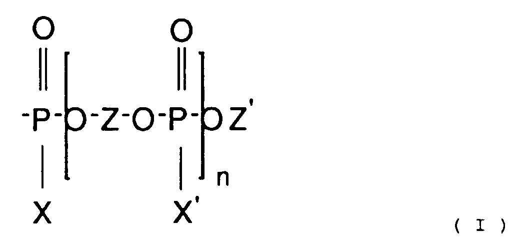 dans laquelle n est zéro ou un nombre entier de 1 à 30,
   les groupements Z sont choisis parmi les groupements alkylène ou alcénylène ayant 2 à 30 atomes de carbone, Z' est un groupement alkyle ou alcényle ayant 2 à 30 atomes de carbone, X et X' représentent indépendamment -O⁻M⁺, -S⁻M⁺ ou un groupement alkyle, alcoxy, aryloxy, aminoalkyle ou aminoalcoxy, M⁺ étant un cation alcalin ou NH₄⁺, étant entendu que l'un au moins des groupements Z et/ou Z' est substitué par un groupement fonctionnel polaire ou par un groupement portant un groupement fonctionnel polaire ;
- les groupements de formule (II) : 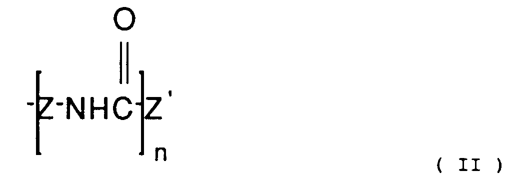 dans laquelle n, Z et Z' sont définis comme précédemment,
- et les groupements de formule (III) :

   -L (III):

   dans laquelle L, est un reste peptidyle (dont la valence libre est liée par exemple à l'extrémité N-terminale ou C-terminale), ledit reste peptidyle comprenant au moins un motif dérivé d'un acide aminé comportant une chaîne latérale substituée par un groupement fonctionnel polaire ;
- ledit ligand répond à la formule (I), n étant zéro ou un nombre entier de 1 à 10, Z est un alkylène ayant de 2 à 12 atomes de carbone, et Z' est un alkyle ayant de 2 à 12 atomes de carbone, l'un au moins des groupements Z et Z' étant substitué par au moins un groupement amine,hydroxy ou hydrazino ; en particulier, n = O et X représente O⁻M⁺ ;
- ledit ligand répond à la formule (II) avec n = O, et Z' représente un groupement alkyle ayant 3 à 12 atomes de carbone, substitué par au moins un groupement amine ; ledit groupement alkyle est en outre substitué par au moins un groupement hydroxy ;
- ledit ligand répond à la formule (II) avec n = 1, Z représente un alkylène ayant au moins 2 atomes de carbone et Z' représente un groupement alkyle substitué par au moins un groupement amine ;
- ledit ligand répond à la formule (III), et ledit acide aminé comportant une chaîne latérale substituée est choisi parmi la lysine, l'arginine, l'ornithine, la glutamine, l'asparagine, la sérine, la thréonine, la tyrosine, l'histidine et le tryptophane.

Le composé de formule générale (III) est par exemple un peptide tel que représenté respectivement par les séquences :
- (KGS)ₘ-KGG,m étant un nombre entier pouvant varier de 1 à 15
- KIEPLGVAPTKAKRRWQREKR

Le terme "fragment d'acide nucléique" tel qu'utilisé dans la présente invention, signifie un fragment d'ADN ou d'ARN naturel ou un oligonucléotide naturel ou de synthèse ou un fragment d'ADN ou d'ARN de synthèse non modifié ou comprenant une ou plusieurs bases modifiées telles que l'inosine, la méthyl-5-désoxycytidine, la diméthylamino-5-désoxyuridine, la désoxyuridine, la diamino-2,6-purine, la bromo-5-désoxyuridine ou toute autre base modifiée permettant l'hybridation, le fragment d'acide nucléique pouvant être simple brin ou double brin.

Le terme "support solide" tel qu'utilisé ici inclut tous les matériaux sur lesquels peut être immobilisé un fragment d'acide nucléique pour une utilisation dans des tests diagnostiques, en chromatographie d'affinité et dans des processus de séparation. Des matériaux naturels, de synthèse, modifiés ou non chimiquement peuvent être utilisés comme support solide , notamment les polysaccharides tels que les matériaux de cellulose, par exemple du papier, des dérivés de cellulose tels que l'acétate de cellulose et la nitrocellulose ; des polymères tels que polychlorure de vinyle, polyéthylène, polystyrènes, polyacrylate ou copolymères tel que polymère de chlorure de vinyle et de propylène, polymère de chlorure de vinyle et acétate de vinyle ; copolymères à base de styrènes ; des fibres naturelles telles que le coton et des fibres synthétiques telles que le nylon.

De préférence, le support solide utilisé dans la présente invention est un polymère de polystyrène, un copolymère butadiènestyrène ou un copolymère butadiène-styrène en mélange avec un ou plusieurs polymères ou copolymères choisis parmi le polystyrène, les copolymères styrène-acrylonitrile ou styrène- méthacrylate de méthyle, les polypropylènes, les polycarbonate ou analogues. Avantageusement, le support de la présente invention est un polystyrène ou un copolymère à base de styrène comprenant entre environ 10 et 90 % en poids de motifs de styrène.

Le support solide selon l'invention peut être, sans limitation, sous la forme d'une plaque de microtitration, d'une feuille, d'un cône, d'un tube, d'un puits, de billes ou analogue.

Le terme "agent de couplage", tel qu'utilisé ici désigne une molécule contenant au moins deux "extrémités" ou groupements réactifs. L'agent de couplage peut être homobifonctionnel ou hétérobifonctionnel.

Les ligands utilisés dans la présente invention, et donnés ici à titre d'exemple, peuvent être des composés disponibles dans le commerce ou des composés synthétisés selon des méthodes connues en soi.

L'invention a également pour objet un support sur lequel est immobilisé par fixation passive un fragment d'acide nucléique (tel que défini précédemment), ce support étant caractérisé par le fait qu'il peut être obtenu selon le procédé décrit ci-dessus. Un tel support peut être utilisé notamment dans diverses techniques telles que celles des tests de diagnostic et celles de la chromatographie d'affinité. L'invention concerne également l'utilisation d'un tel support dans un procédé de détection ou de purification, selon les techniques connues, d'une cible nucléotidique contenant une séquence complémentaire de celle du fragment d'acide nucléique immobilisé : en particulier, ce fragment immobilisé peut être employé comme sonde de capture.

Les exemples suivants illustrent l'invention sans toutefois la limiter.

### Exemple 1 : ligands phosphoramidites :

Les composés disponibles dans le commerce utilisés dans la présente invention sont listés dans le tableau 1 ci-après

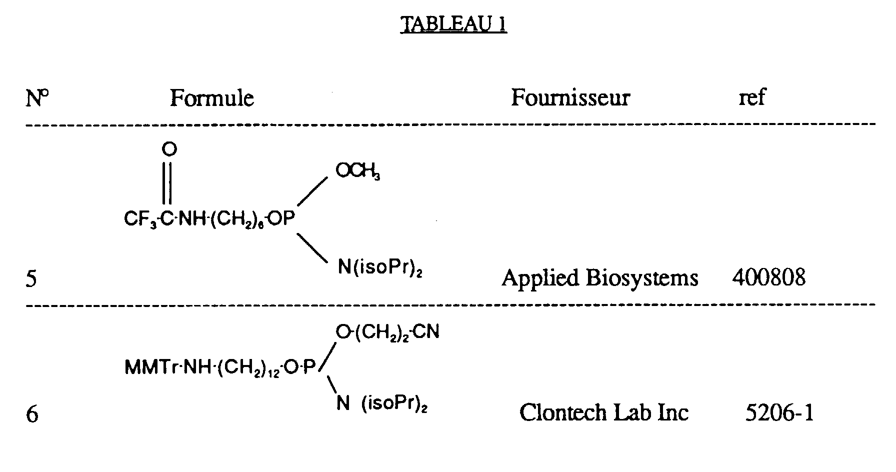

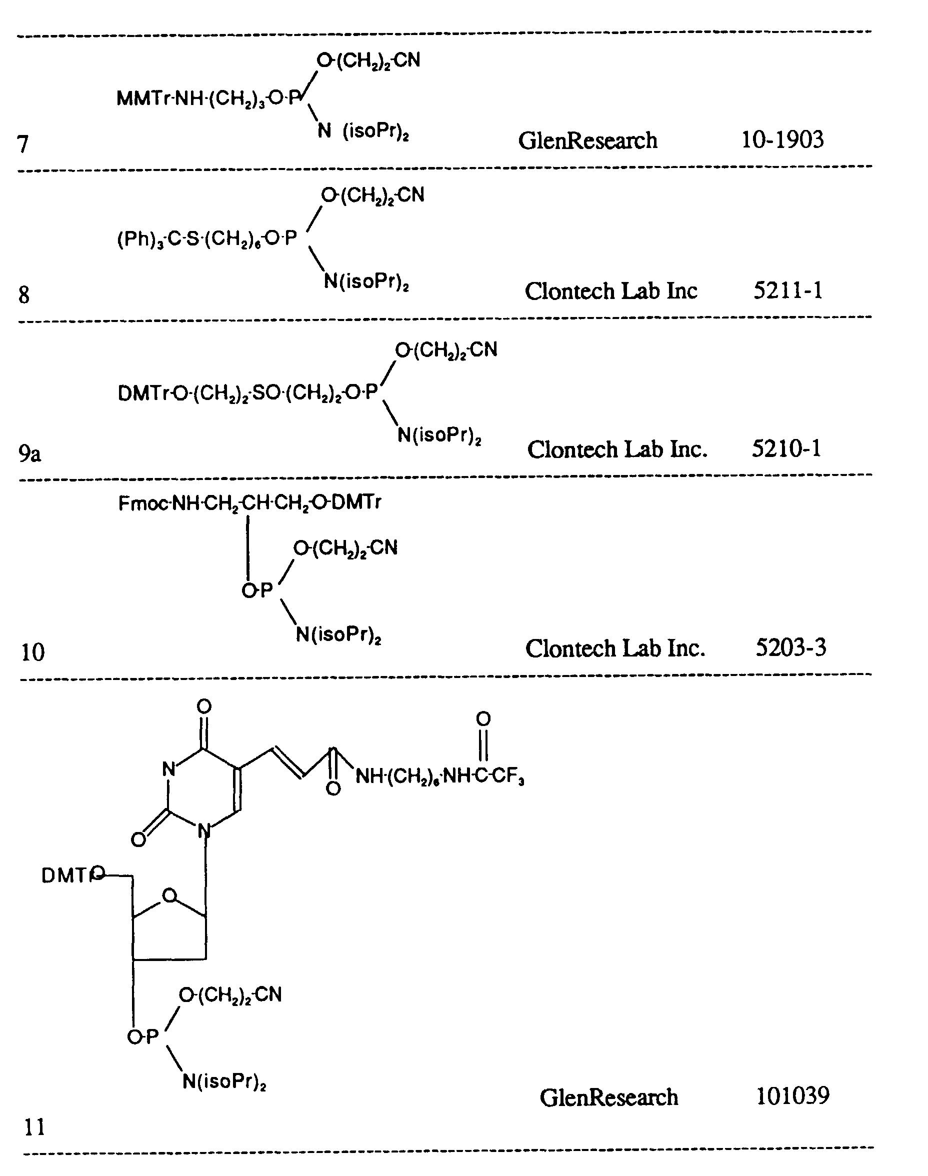

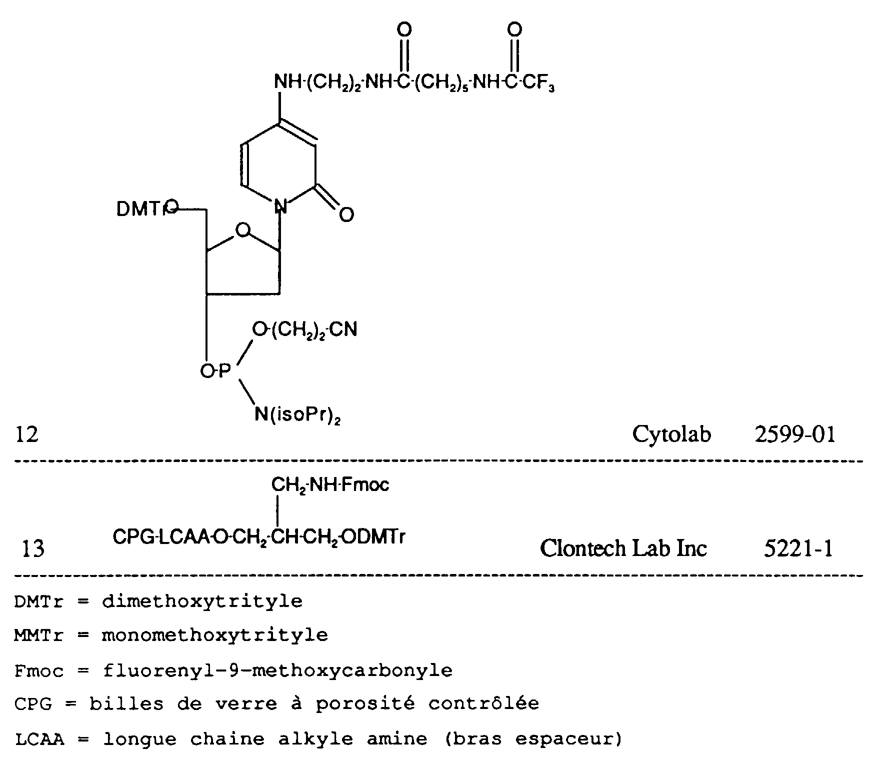

D'autres ligands phosphoramidites préparés par la demanderesse sont respectivement synthétisés selon les protocoles décrits ci-dessous :
a) - Dans un ballon de 100 ml purgé à l'argon sont mélangés 2 g d'octanediol-1,8 (commercialisé par la société ALDRICH 0-330-3), 0,084 g de diméthyl-4 aminopyridine, 1,9 ml de triethylamine dans 20 ml de pyridine anhydre. Puis 3 g de chlorure de diméthoxy-4,4' triphenylméthyle dans 15 ml de pyridine sont additionnés en 30 min. Après 1 heure de réaction sous agitation, la réaction est bloquée par 10 ml de méthanol. Après extraction par un mélange acétate d'éthyle, tampon phosphate de sodium 0,1 M à pH 7, la phase organique est séchée sur sulfate de sodium, puis purifiée sur colonne de silice par un mélange de CH₂Cl₂/méthanol/triethylamine selon les proportions suivantes : 98/1/1.
   Les fractions contenant un produit dont le facteur de rétention (Rf) est égal à 0,47 sont mélangées et concentrées sous vide. 0,7 g de ce produit séché sont mis en solution dans 4 ml de CH₃CN anhydre avec 4,7 ml de tétrazole à 0,5 M dans CH₃CN. La solution est ajoutée goutte à goutte dans 0,5 g de cyanoethyl-2 N,N,N',N'-tétraisopropylphosphoro-diamidite (commercialisé par la société ALDRICH sous la référence 30-599-5) dans 6 ml de CH₃CN.
   Après 30 minutes de réaction, la phase organique est centrifugée. Le surnageant est purifié par chromatographie sur couche mince sur colonne de silice en utilisant un mélange A composé d'hexane/acétate d'éthyle/triéthylamine respectivement selon les proportions 60/35/5. Les solutions contenant le produit souhaité sont regroupées et concentrées.
   Le composé obtenu est le composé référencé 1 représenté par la formule générale IV ci-dessous dans laquelle DMTr représente le diméthoxytrityle. 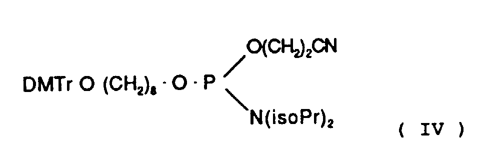
   Le déplacement chimique en RMN (résonance magnétique nucléaire) du ³¹P du composé 1 est de 148,40 ppm et son facteur de rétention (Rf) dans le mélange A est de 0,77. Les déplacements chimiques en RMN sont donnés par rapport à H₃PO₄ à 85 %.
b) - 210 mg de benzyloxy-2-éthanol (commercialisé par la société ALDRICH sous la référence 25286-7) anhydre dans 10 ml de tétrazole à 0,25 M dans CH₃CN sont additionnés goutte à goutte dans 3 ml de CH₃CN contenant 600 mg de cyano-2 N,N,N',N'-tétraisopropyl phosphorodiamidite.
   Après 1 heure de réaction sous agitation, centrifugation pour éliminer le précipité, la phase organique est concentrée et purifiée par chromatographie sur couche mince sur colonne de silice par un mélange B hexane/acétate d'éthyle/triethylamine respectivement selon les proportions 50/45/5.
   Le composé obtenu est le composé référencé 2 représenté par la formule générale V ci-dessous. 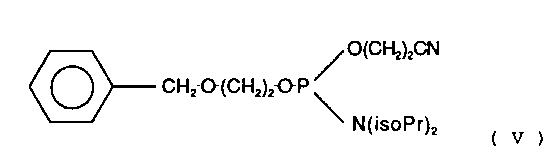 Les caractéristiques du composé 2 sont les suivantes :
   RMN ³¹P (ppm) = 148,57, Rf = 0,85 dans le mélange B.Les déplacements chimiques en RMN sont donnés par rapport à H₃PO₄ à 85%.
c) - On utilise un protocole identique à celui décrit pour la synthèse du composé 2 en utilisant comme produit de départ du phényl-6-hexanol-1 (commercialisé par la société ALDRICH sous la référence 33361-1).
   Après 1 heure de réaction sous agitation, centrifugation pour éliminer le précipité, la phase organique est concentrée et purifiée par chromatographie sur couche mince sur colonne de silice en utilisant un mélange C composé d'hexane/acétate d'éthyle/triéthylamine respectivement selon les proportions 70/25/5.
   Le composé obtenu est le composé référencé 3 représenté par la formule générale VI ci-dessous : 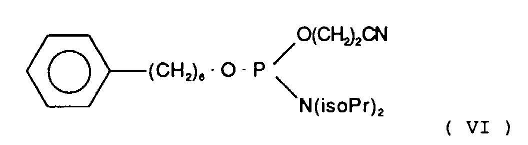 Les caractéristiques du composé 3 sont les suivantes :
   RMN ³¹P (ppm) = 148,40, Rf = 0,70 dans le mélange C.
d) - On utilise un protocole de synthèse identique à celui décrit pour le composé 2 en utilisant comme produit de départ l'hexanol-1 (commercialisé par la société ALDRICH sous la référence H 1330-3). La réaction n'est pas suivie par chromatographie sur couche mince, mais par RMN du ³¹P. La réaction est stoppée après disparition du bisphosphoramidite de départ (δ³¹P = 128,18 ppm). Le composé obtenu est le composé référencé 4 représenté par la formule générale VII ci-dessous et est utilisé brut après filtration sur Millex (désignation commerciale) 0,45 µm. 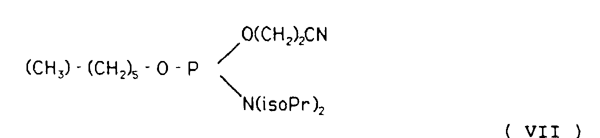 Le déplacement chimique en RMN du ³¹P est égal à 148,37 ppm.
e) On utilise un protocole de synthèse identique à celui décrit pour le composé 2 en utilisant comme produit de départ le dimethylamino-3 propanol-1 (commercialisé par la société ALDRICH sous la référence D14440-1). La réaction n'est pas suivie par chromatographie sur couche mince, mais par RMN du ³¹P. La réaction est stoppée après disparition du bisphosphoramidite de départ (δ³¹P = 128,18 ppm). Le composé obtenu peut être utilisé brut après filtration sur Millex (désignation commerciale) 0,45 µm.
f) On utilise un protocole de synthèse identique à celui décrit pour le composé 2 mais en utilisant comme produit de départ un N-methyl aminoalcool tel que le (methylamino)-2 ethanol(commercialisé par la société ALDRICH sous la référence 23966-6).

### EXEMPLE 2 : Synthèse d'un bras thiophosphate à l'extrémité 5' de l'oligonucléotide

Une solution de 15,02 g d'hydroxy-3 proprionitrile (10 992-4 Aldrich) dans 40 ml de THF anhydre et 26 ml de N, N'- diisopropyléthylamine (Aldrich D 12580-6) est refroidie à - 10° C. 10,10 g de dichloro(diisopropylamine)phosphine sont additionnés sous agitation en 30 min. Après 20 min supplémentaires de réaction, le chlorhydrate est filtré sous atmosphère inerte. Le filtrat dilué dans 1 litre d'acétate d'éthyle est extrait 3 fois avec 150 ml de Tampon phosphate pH 7,00 puis séché sur Na₂SO₄. Après concentration sous vide, le produit est purifié sur silice en présence de triethylamine.

Le composé obtenu est le composé référencé 9b représenté par la formule VIII ci-dessous.

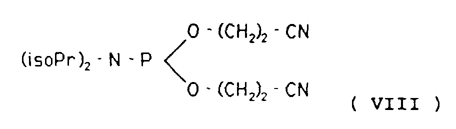

### Exemple 3 : Ligands peptidiques

Les peptides sont synthétisés sur une résine phénylacétamidomethyl (PAM) polystyrène/divinylbenzène (Applied Biosystems, Inc. Foster City, CA) par la méthode en phase solide de Merrifield en utilisant un synthétiseur automatique Applied Biosystems 430A. Les acides aminés sont couplés sous forme d'anhydride symétrique à l'exception de la glutamine et de l'asparagine qui réagissent sous forme d'esters d'hydroxybenzotriazole (HOBT). Les acides aminés utilisés proviennent de chez Novabiochem (Laüflerlfingen, Suisse) ou de chez BACHEM (Bubendorf Suisse).

La synthèse chimique des peptides a été réalisée en utilisant un protocole de double couplage avec la N-methyl-pyrrolidone (NMP) comme solvant. Les peptides ont été coupés de leur résine ainsi que les protections latérales de manière simultanée à l'aide d'acide fluorhydrique (HF) dans un appareillage approprié (HF appareil de coupure de Type I, Peptide Institute, Osaka, Japon).

Pour 1 g de peptidylrésine, 10 ml de HF, 1 ml d'anisole et 1 ml de dimethylsulfure (DMS) sont utilisés et le mélange est agité durant 45 minutes à - 2° C. Le HF est alors évaporé sous vide. Après des lavages intensifs à l'éther, le peptide est élué de la résine par de l'acide acétique 10 % puis de l'acide acétique concentré. Après dilution par de l'eau le peptide est lyophilisé.

Les peptides sont purifiés par chromatographie liquide haute performance préparative sur une colonne VYDAC de type C18 (250 x 21 mm) (The Separation Group, Hesperia, CA, U.S.A). L'élution est réalisée par un gradient d'acétonitrile à un débit de 22 ml/min. Les fractions collectées sont contrôlées par une élution en condition isocratique sur une colonne VYDAC C18 analytique (250 x 4,6 mm) à un débit de 1 ml/min. Les fractions qui présentent le même temps de rétention sont réunies et lyophilisées. La fraction majoritaire est ensuite analysée par CLHP analytique avec le système décrit précédemment. Le peptide qui est considéré comme de pureté acceptable se traduit par un pic unique représentant 95 % minimum du chromatogramme.Les peptides purifiés sont alors analysés dans le but de contrôler leur composition en acides aminés. Il sont tout d'abord hydrolysés dans de l'HCl 6N (Pierce) additionné de phénol (1% final) à 110° C durant 24 h sous vide dans une station d'hydrolyse Picotag (WATERS, Millipore Corporation, Milford USA). L'analyse des acides aminés est ensuite effectuée à l'aide d'un analyseur d'acides amines BECKMAN 6300. La mesure de la masse moléculaire chimique (moyenne) des peptides est obtenue en utilisant la spectrométrie de masse L.S.I.M.S. en mode d'ion positif sur un instrument à double focalisation VG. ZAB.ZSEQ relié à un système d'acquisition DEC-VAX 2000 (VG analytical Ltd, Manchester, Angleterre).

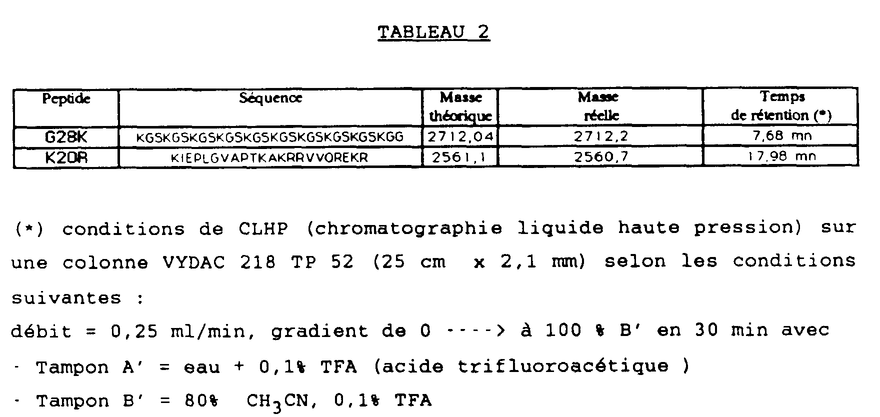

### Exemple 4 : couplage d'un ligand phosphoramidite.

Le couplage d'un ligand phosphoramidite à un oligonucléotide est effectué selon le protocole général suivant :

Un oligonucléotide est synthétisé sur un appareil automatique APPLIED 381 A de la société APPLIED BIOSYSTEMS en utilisant la chimie des phosphoramidites selon le protocole du constructeur. Le ligand phosphoramidite dissout dans de l'acétonitrile anhydre à une concentration de 0,2 M, est placé en position X du synthétiseur et l'addition du ligand se fait à l'extrémité 5' de l'oligonucléotide selon le protocole standard de la synthèse automatique lorsque la synthèse de l'oligonucléotide est achevée.Dans le cas où le ligand est porteur d'un groupement protecteur dimethoxytrityle, tel que pour les composés 1 et 9 à 12 décrits précédemment, il est nécessaire d'effectuer une étape supplémentaire de déprotection du groupement trityle par l'acide trichloroacétique en fin de synthèse. Après déprotection une nuit à 55°C dans NH₄OH 33 %, précipitation dans de l'éthanol à -20°C, l'oligonucléotide est séché sous vide et repris dans 1 ml d'eau.

Pour les composés référencés 6 et 7, une étape supplémentaire de clivage du groupement monomethoxytrityle est effectuée selon le protocole du fabricant (CLONTECH) après déprotection.

Pour les composés 11 et 12, le couplage est effectué selon le protocole général décrit ci-dessus jusqu'à la déprotection, mais le ligand est greffé sur la base terminale de l'extrémité 5' de l'oligonucléotide.Il faut noter par ailleurs que les ligands 1, 10, 11 et 12 peuvent s'additionner sur eux-mêmes par l'intermédiaire du groupement hydroxyle libéré par detritylation.
Dans le cas du composé portant la référence 13, la synthèse automatique démarre par la silice greffée par le ligand sur le protocole standard. Le couplage ligand et oligonucléotide s'effectue par l'extrémité 3' de ce dernier.

Il est dans les connaissances de l'homme de l'art de fabriquer le support silice avec les ligands convenablement protégés pour la synthèse automatique ou pouvant être déprotégés comme expliqué précédemment.

Dans tous les cas, les oligonucléotides modifiés à leurs extrémités 5' ou 3' sont purifiés par chromatographie liquide haute pression en phase inverse sur une colonne Brownlee RP18 (10 mm - 25 cm), effectuant un gradient de 10 à 35 % de Tampon B" en 30 minutes puis de 35 à 100 % de Tampon B" en 3 minutes: Les caractéristiques des tamponsA" et B" sont les suivantes :
- Tampon A" = 0,1 M TEAA (triethylammoniumacétate), pH = 7,0
- Tampon B" = 50 % A + 50 % CH₃CN

### Exemple 5 : Couplage d'un ligand thiophosphate à un oligonucléotide

Une solution 0,2 M du composé 9b dans CH₃CN est additionnée, tel que précédemment décrit dans l'exemple 4, à l'extrémité 5' d'un oligonucléotide en supprimant l'étape d'oxydation par le mélange I₂, H₂O, THF, pyridine. La cassette comportant la silice greffée par l'oligonucléotide est mise en contact 1 heure avec une solution de soufre à 5 % dans un mélange CS₂/Pyridine respectivement selon les proportions 50/50. Après rinçage soigneux par le mélange CS₂/pyridine, une déprotection est effectuée comme décrit précédemment.

Le tableau 3 ci-dessous illustre les produits obtenus après couplage des composés 1 à 13 précédemment décrits sur un oligonucléotide quelconque et déprotection, et donne également à titre de référence un oligonucléotide nu.

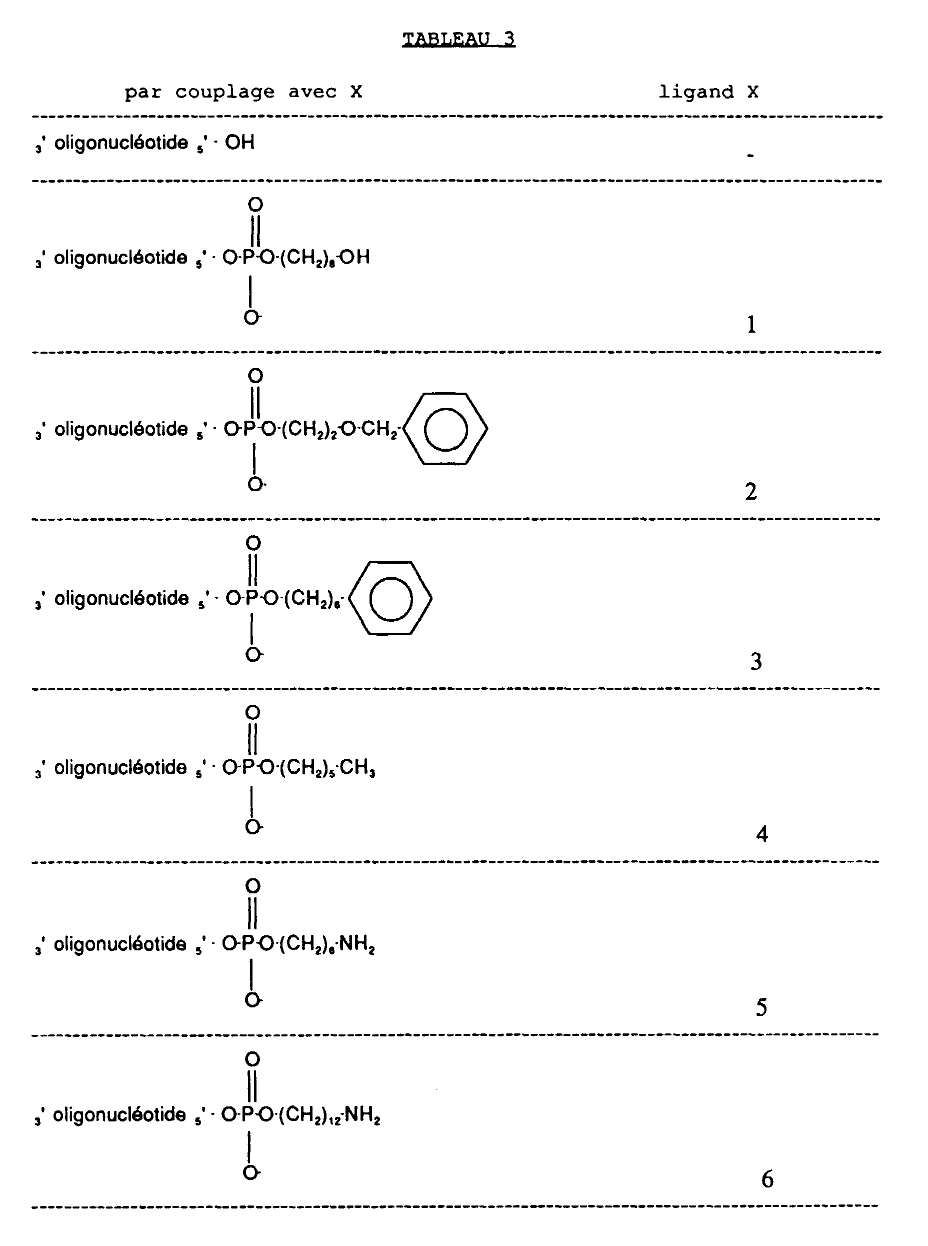

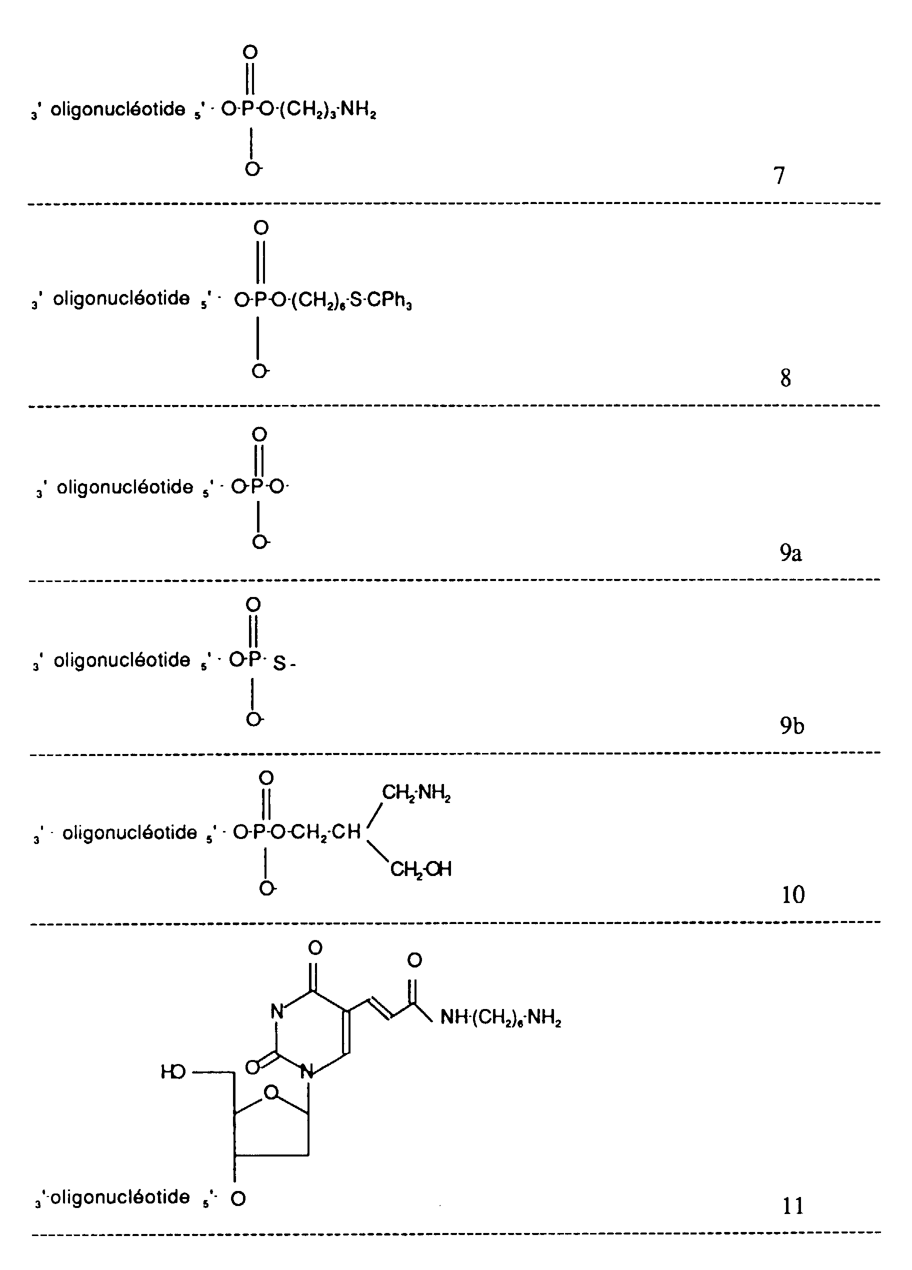

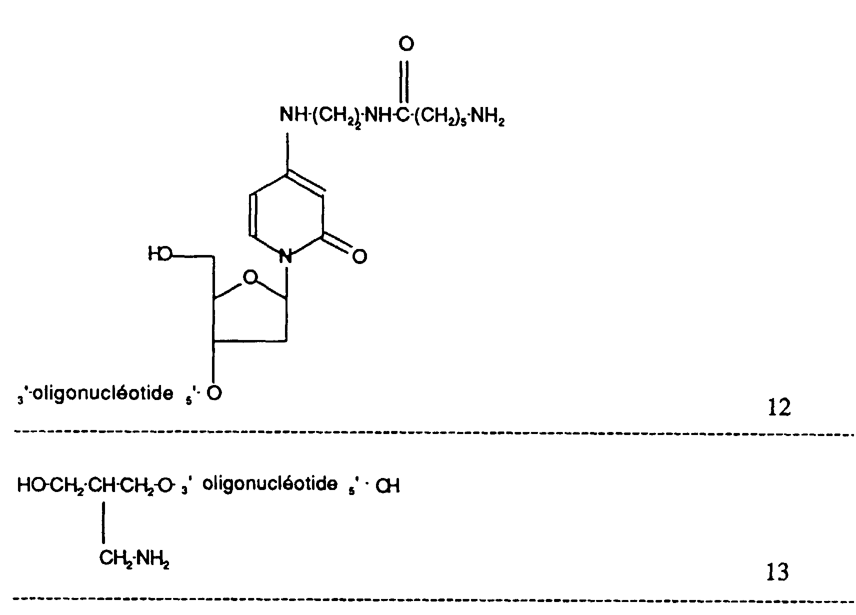

### Exemple 6 : couplage d'un ligand peptidique à un oligonucléotide

Le couplage d'un peptide à un oligonucléotide peut être effectué selon les méthodes dites directes ou indirectes connues. Par méthode directe, on entend le couplage par synthèse automatique telle que décrite dans la publication de C. D. Juby, Tetrahedron Lett., 879 (1991) et la méthode selon laquelle on synthétise un oligonucléotide ayant une fonction réactive à son extrémité 5' ou 3' ou sur une base. On fait subir à l'oligonucléotide une étape de déprotection et on le couple à un peptide, préparé au préalable, par formation d'une liaison covalente entre deux fonctions réactives complémentaires, l'une portée par l'oligonucléotide et l'autre par le peptide. Par exemple, on peut coupler des amines primaires avec un acide carboxylique activé ou un aldéhyde ou bien une fonction thiol avec un halogénoalkyle. Méthode de couplage indirecte signifie que l'oligonucléotide et le peptide sont chacun porteurs d'une fonction réactive identique ou différente l'une de l'autre, ces 2 fonctions n'étant pas complémentaires. En conséquence, il est nécessaire d'utiliser un agent intermédiaire de couplage qui peut être homobifonctionnel si les deux fonctions sont identiques ou hétérobifonctionnel si les deux fonctions sont différentes. Parmi les agents de couplage homobifonctionnels, on peut citer le DITC (phénylène-1,4-diisothiocyanate) lorsque les deux fonctions réactives sont des fonctions amines primaires, le DSS (disuccinimidylsubérate) ou analogues. Parmi les agents de couplage hétérobifonctionnels, on peut citer le SMCC (succinimidyl-4-(N-maléimidométhyl)cyclohexane-1-carboxylate) lorsque les deux fonctions réactives présentent chacune indépendamment de l'autre une fonction amine primaire et une fonction thiol, le SMPB (succinimidyl-4-(p-maléimido phényl) butyrate ) ou analogues.

Selon l'invention, on a réalisé le couplage d'un oligonucléotide et d'un peptide par technique indirecte telle que décrite en détail ci-après. Mais bien entendu, ceci n'est qu'une illustration non limitative d'un mode de réalisation particulier de l'invention.

Aussi, selon l'invention, on arrive à synthétiser un oligonucléotide avec un ligand représenté par le composé 5, puis on procède à une étape de purification comme décrit dans l'exemple 4. Après séchage sous vide, l'oligonucléotide (3.10⁻⁸ moles) a été repris dans 25 µl de Tampon borate de sodium 0,1 M pH 9,3. On a additionné 500 µl à 30 mg/ml de DITC (Fluka 78 480) dans le DMF. Le mélange a été agité 1 heure 30 min environ à 37°C avant addition de 3 ml d'H₂O.

Après extraction de la solution par le butanol (3 x 3 ml), la phase aqueuse restante (600 µl) est séchée sous vide, puis reprise par 1,5 10⁻⁷ moles de peptides dans 300 µl de tampon borate 0.1M pH 9,3. Après une nuit de réaction à 37°C sous agitation, le conjugué oligopeptide obtenu a été purifié sur colonne Brownlee RP 300 (4,6 x 100 mm) dans les conditions suivantes :

On a utilisé deux tampons respectivement C - 0.1 M TEAA, pH 8,0 et D = 50 % C + 50 % CH₃CN. On a réalisé un gradient de 0 à 30 % de C en 30 min, puis de 30 à 100 % de C en 3 min.

En utilisant les protocoles décrit précédemment, on a respectivement synthétisé 3 oligonucléotides a, b et c et couplés ou non à divers ligands phosphoramidites, thiophosphates ou peptidiques.

La séquence nucléotidique de l'oligonucléotide a est la suivante :

5'-GCATTTAGAGCC-3'.

L'oligonucléotide b est représenté par la séquence :

5'-TGCATTTAGAGCC-3'.

L'oligonucléotide c est caractérisé par la séquence

5'-TCTACGCATTTCACCGCTACAC-3'.

Les résultats sont présentés dans le tableau 4 ci-après

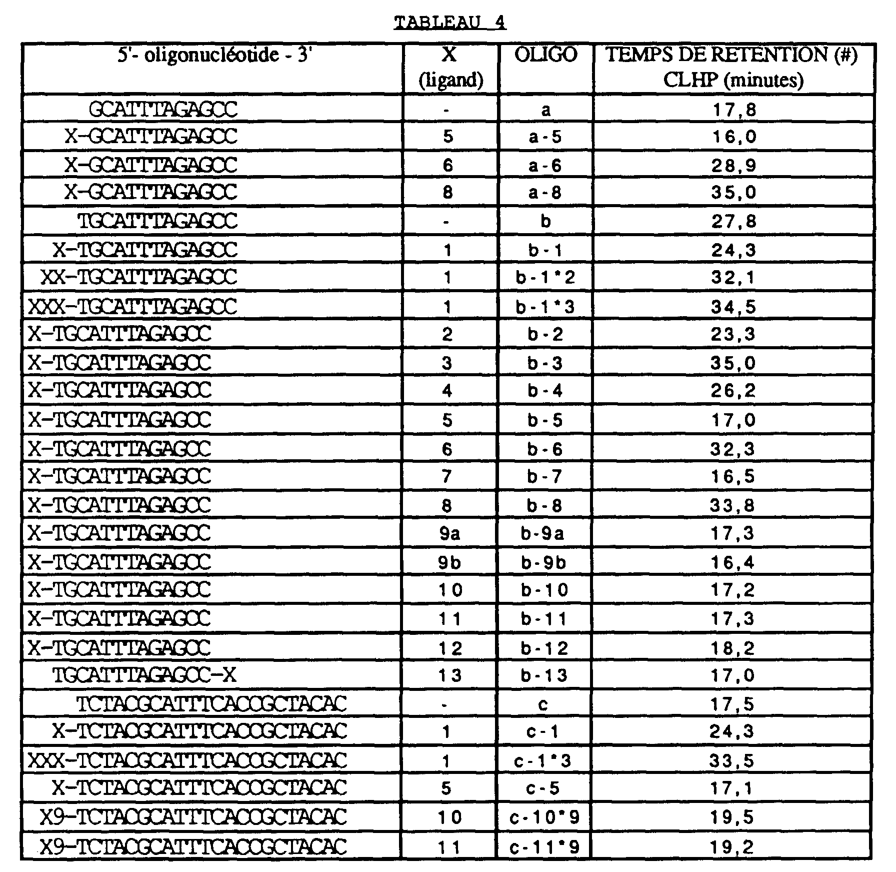

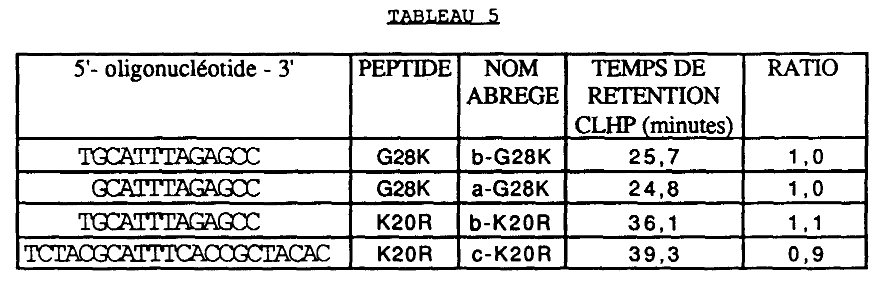

dans lequel X caractérise le ligand selon la nomenclature utilisée précédemment, le symbole ∗n, n représentant le nombre 2, 3 ou 9, signifie que le ligand peut s'additionner n fois sur lui-même.

Le symbole - dans la colonne X du tableau 4 signifie que l'oligonucléotide est synthétisé sans ligand.

Le symbole # signifie que les conditions chromatographiques sont celles décrites pour l'exemple 4.

Dans le tableau 5, l'oligonucléotide est quantifié en picomoles par U.V. en mesurant l'absorbance à 260 nm, selon le protocole Applied Biosystems. Le peptide est quantifié en picomoles par analyse d'acides aminés selon la méthode décrite dans l'exemple 3. Le ratio oligo/peptide est le rapport de ces deux valeurs.

### Exemple 7 : détection d'un fragment d'acide nucléique par la technique dite "sandwich".

Dans une plaque de microtitration en polystyrène (Nunc 439454), sont déposés 100 µl d'une solution d'un oligonucléotide de capture modifié ou non par un ligand à une concentration de 0,15 µM dans du PBS 3X (0,45 M NaCl; 0,15 M phosphate de sodium,; pH 7,0). La plaque est incubée deux heures à environ 37°C puis lavée 3 fois par 300 µl de PBS Tween® (0,15 M NaCl; 0,05 M phosphate de sodium; pH 7,0; 0,5% Tween® 20 (MERCK 822184)).

50 µl d'une séquence cible à différentes concentrations dans du tampon PBS saumon (PBS 3X + ADN de sperme de saumon 10 mg/ml (Sigma D9156)) sont ajoutés dans les puits, suivis de 50 µl d'une solution d'un conjugué oligonucléotide-peroxydase à une concentration de 0,1 ng/µl (15 nM) en oligonucléotides dans un tampon PBS - cheval (PBS 3X + 10 % de sérum de cheval (BIOMERIEUX 55842)).

La plaque est incubée 1 heure à 37°C et lavée par 3 fois 300 µl de PBS Tween®.

100 µl de substrat OPD (ortho-phénylène diamine, Cambridge Medical Biotechnology ref. 456), dans un tampon OPD (0,05 M acide citrique; 0,1 M NaH₂PO₄; pH 4,93) de la concentration de 4 mg/ml, auxquels on ajoute extemporanément H₂O₂ à 30 volumes au 1/1000, sont ajoutés par puits. Après 20 minutes de réaction, l'activité enzymatique est bloquée par 100 µl d'H₂SO₄ 1N. La lecture est effectuée sur un lecteur AXIA Micro Reader (BIOMERIEUX) à 492 nm.

### Exemple 8 :

Conformément au protocole général décrit dans l'exemple 7, on a effectué la détection d'un fragment d'acide nucléique cible déterminé par la séquence 
5'-AAGGTCAACCGGAATTTCATTTTGGGGCTCTAAATGCAATACAATGTCTTG
CAATGTTGCCTTA-3',
 respectivement à une concentration de 10⁻⁹ M et de 10⁻¹⁰ M en utilisant un oligonucléotide de capture de séquence 5'-TGCATTTAGAGCC-3' modifié ou non par un ligand et un oligonucléotide de détection conjugué à la peroxydase de séquence

5'-TAAGGCAACATTGCAAGATA-3'.

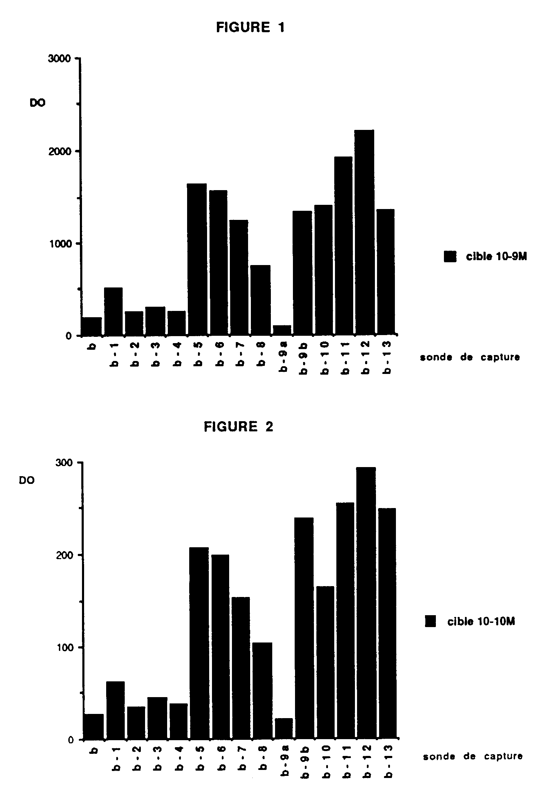

Les résultats sont présentés aux figures 1 et 2 dans lesquelles les oligonucléotides de capture sont notés selon les règles définies précédemment dans les tableaux 4 et 5.

L'effet de l'addition d'un ligand à l'extrémité d'un oligonucléotide sur l'efficacité de la détection peut se quantifier par le ratio signal oligonucléotide-ligand/signal oligonucléotide nu. On entend par oligonucléotide nu, un oligonucléotide de synthèse standard, c'est-à-dire 3' OH, 5' OH et ne possédant pas de ligand sur les bases.

On a représenté dans le tableau ci-dessous le ratio signal oligonucléotide-ligand/signal oligonucléotide nu, dans lequel X représente un ligand selon la terminologie utilisée précédemment et b représente un oligonucléotide tel que déterminé précédemment.

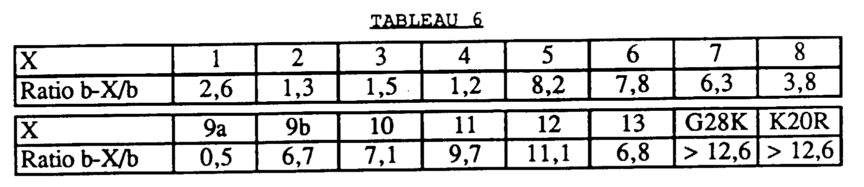

Ce tableau montre que l'addition d'un ligand sur un oligonucléotide permet d'augmenter de manière importante le signal, jusqu'à plus de 10 fois dans certains cas. Tous les ligands n'ont pas le même effet et la présence d'une amine primaire est un facteur déterminant. Ceci est d'ailleurs confirmé par les figures 1 et 2 (donnant les résultats obtenus (donnés en D.O. (densité optique)) avec diverses sondes de capture).

Comme cela ressort des figures 1 et 2, il n'y a pas d'effet de la concentration en cible.

Avec les ligands 5, 6, 7, 10 et 13 qui comportent une amine primaire sur une chaîne alkyle ramifiée ou non, le ratio b-X/b est compris entre 6,3 et 8,2, alors que pour les ligands 1, 2, 3, 4, 9 qui ne comportent pas d'amines primaires, ce ratio est compris entre 1,2 et 3,7.

Dans le cas du ligand 9a, qui amène un groupement phosphate, l'effet est négatif avec un ratio de 0,5.

Si le ligand aminé est porté par une chaîne aliphatique greffée sur la base d'un nucléotide non complémentaire de la cible, le même effet d'augmentation du signal est visible, ce qui est le cas avec les ligands 11 et 12.

### Exemple 9 :

On répète le protocole général décrit dans l'exemple 7 utilisant des ligands simples, des ligands pouvant s'additionner sur eux-mêmes, des ligands peptidiques et un nucléotide thymine (T). L'oligonucléotide de capture b est caractérisé par la séquence 5'-TGCATTTAGAGCC-3'.

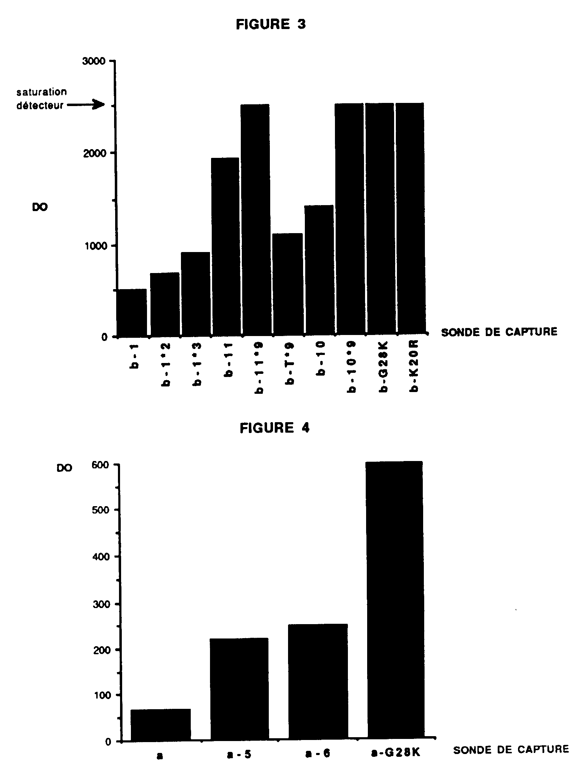

Les résultats sont présentés à la figure 3 et dans le tableau 7.

**TABLEAU 7**

| X | 1 | 1*2 | 1*3 | 11 | 11*9 | T*9 | 10 | 10*9 | G28K | K20R |
|---|---|---|---|---|---|---|---|---|---|---|
| Ratio b-X/b | 2,6 | 3,4 | 4,6 | 9,7 | >12,6 | 4,4 | 7,1 | >12,6 | >12,6 | >12,6 |

Comme cela ressort de cette figure et du tableau 7, dans lequel X représente le ligand, le symbole ∗n signifie le ligand pouvant d'additionner n fois sur lui-même, b représente l'oligonucléotide de capture.

La sensibilité de détection est augmentée par l'addition multiple du ligand, quel que soit le type de ligand, mais avec un effet très marqué lorsque le ligand est porteur d'une fonction amine. Cet effet de la fonction amine est confirmé par la comparaison entre ligand 1 ((CH₂)₈-OH) et le ligand 5 ((CH₂)₆-NH₂) de l'exemple précédent. En effet, le ligand 1 porteur d'un groupement hydroxyle, même additionné 3 fois sur lui-même, ne donne un gain que de 4,56, alors que le ligand 5 portant un groupement amine primaire produit un gain de 8,20. De même pour le ligand thymine (T) additionné 9 fois sur lui-même, le gain de sensibilité n'est que de 4,4 en comparaison avec le ligand 11 additionné 9 fois sur lui-même, qui donne un gain de sensibilité de 12,6. Le ligand 11 diffère du ligand thymine par la présence d'un bras aliphatique comportant une amine primaire sur la base T en position 5.

### Exemple 10 :

On utilise le même protocole général que celui décrit dans l'exemple 7. On utilise une sonde de capture courte, référencée a, de séquence 5'-GCATTTAGAGCC-3' et qui est raccourcie d'une base par rapport à la sonde b précédemment testée. Les ligands couplés à la sonde de capture sont respectivement les ligands phosphoramidite 5 et 6 et le ligand peptidique G28K.

La cible est déposée à une concentration égale à 10⁻¹⁰ M.

Comme cela ressort à la figure 4, la sensibilité de détection est augmentée en présence d'un ligand porteur d'une fonction amine primaire, par rapport à un oligonucléotide nu. Par ailleurs, on constate que la sensibilité de détection est fortement augmentée en présence d'un ligand peptidique en comparaison avec un ligand phosphoramidite 5 et 6. Les résultats sont confirmés dans le tableau 8 ci-dessous, dans lequel X représente le ligand, a l'oligonucléotide.

**TABLEAU 8**

| X | 5 | 6 | G28K |
|---|---|---|---|
| Ratio a-X/a | 3,2 | 3,7 | 8,9 |

### Exemple 11 :

On utilise un protocole de détection identique à celui décrit dans l'exemple 7. L'oligonucléotide de capture est l'oligonucléotide c de séquence 5'-TCTACGCATTTCACCGCTACAC-3' modifié à l'extrémité 5' par un ligand et qui est déposé à une concentration de 0,30 µM. La cible est composée de 5.10⁹ Moles d'un mélange d'ARN 16S et 23S E. Coli (commercialisé par la société BOEHRINGER sous la référence 206938) dans 50 µl d'un tampon PBS saumon. La sonde de détection couplée à la peroxydase a pour séquence 5'-TCTAATCCTGTTTGCTCCC-3'.

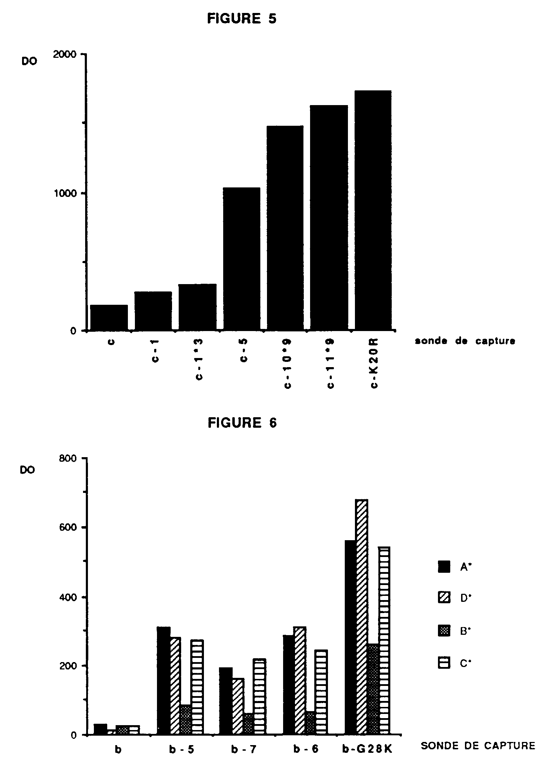

Comme montré à la figure 5, la sensibilité de détection est augmentée par la présence d'un ligand et ceci d'autant plus qu'on utilise un ligand couplé plusieurs fois sur lui même.

Les résultats sont confirmés dans le tableau 9 ci-dessous, dans lequel X représente le ligand et c l'oligonucléotide de capture.

**TABLEAU 9**

| X | 1 | 1*3 | 5 | 10*9 | 11*9 | K20R |
|---|---|---|---|---|---|---|
| Ratio c-X/c | 1,5 | 1,8 | 5,7 | 8,2 | 9,1 | 9,6 |

On constate par ailleurs qu'avec une sonde de capture de 22 bases, les ligands 10 et 11 additionnés 9 fois sur eux-mêmes et le ligand peptidique K20R ont sensiblement le même effet.

### Exemple 12 :

On utilise le même protocole que celui décrit dans l'exemple 7. On fait varier la nature du tampon de dilution de l'oligonucléotide de capture modifié par des ligands chimiques. L'oligonucléotide de capture est l'oligo b tel que décrit précédemment, mis en solution à 0,15 µM respectivement dans des tampons A*, B*, C*, D*. La cible S décrite dans l'exemple 8 est déposée à une concentration de 10⁻¹⁰ M.
A* = 150 mM phosphate de sodium pH 7,0 ; 450 mM NaCl
B* = 150 mM phosphate de sodium pH 4,2 ; 450 mM NaCl
C* = 150 mM phosphate de sodium pH 8,5 ; 450 mM NaCl
D* = 150 mM Tris pH 7,0 ; 450 mM NaCl.

Comme cela ressort de la figure 6, quel que soit le tampon, l'effet du ligand sur la sensibilité de détection est conservé. Cet effet diminue à pH acide avec le tampon B∗.

### Exemple 13 :

On va montrer maintenant l'effet du ligand sur la spécificité. On utilise un protocole sandwich identique à celui décrit dans l'exemple 7 et 2 fragments d'ADN à une concentration de 10⁻¹⁰ M comme cible. La séquence S ci-dessous est parfaitement homologue de l'oligonucléotide de capture b modifié ou non par des ligands .

La séquence SGT décrite ci-dessous contient un mésappariement de type GT au milieu de la région complémentaire de la sonde de capture.

Séquence S =
3'-ATTCCGTTGTAACGTTCTGTAACATAACGTAAATCTCGGGGTTTTACTTTAAGGCCA
ACTGGAA-5'

Séquence SGT =
3'-ATTCCGTTGTAACGTTCTGTAACATAACGTAAGTCTCGGGGTTTACTTTAAGGCCA
ACTGAA-5'

Le gain en signal amené par la présence d'un ligand ne doit pas diminuer la spécificité de la détection. Le ratio signal S/signal SGT peut donc servir de référence pour mesurer les performances du système.

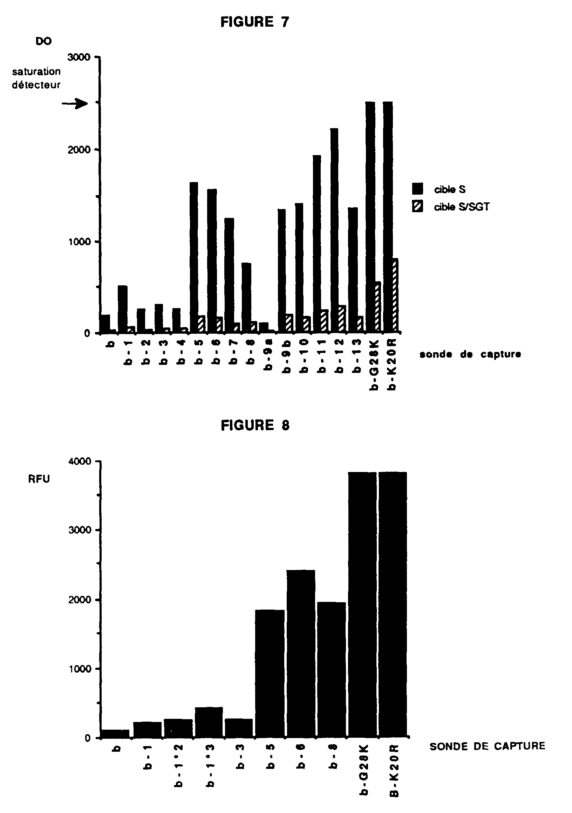

Les résultats sont présentés à la figure 7 et dans le tableau 10 ci-dessous, dans lequel b représente l'oligonucléotide de capture et X le ligand.

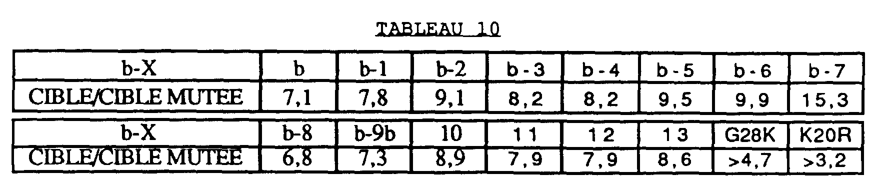

On constate que le gain en signal amené par le ligand ne diminue en rien la spécificité de la détection. Dans le cas de l'utilisation des ligands peptidiques, on note une diminution de gain en signal, en comparaison de celui obtenu avec des ligands phosphoramidites et ceci est dû à une saturation du détecteur. En réalité, la spécificité de la détection n'est pas altérée par l'utilisation du ligand peptidique.

### Exemple 14 :

On réalise la détection d'un fragment d'acide nucléique sur un automate VIDAS (marque enregistrée - commercialisé par la société BIOMERIEUX SA - VITEK). La détection est effectuée à l'aide d'un support conique SPR (commercialisé par la société VITEK (USA),marque de commerce) réalisé à partir d'un matériau vendu sous la dénomination K résine(qui est un copolymère butadiène-styrène) et d'une barrette. La réaction d'hybridation sandwich décrite dans le protocole ci-dessous se produit sur la paroi interne du cône.

Sur la surface interne du cône SPR, sont fixés passivement des oligonucléotides modifiés ou non par un ligand à une concentration de 0,15 µM dans un volume de 315 µl d'une solution de PBS 4X (phosphate de sodium 200 mM pH 7,0, NaCl 600 mM). Après une nuit à température ambiante ou deux heures à 37°C, les cônes sont lavés 2 fois par une solution de PBS Tween, puis séchés sous vide. La barrette contient l'ensemble des réactifs nécessaires à la détection, c'est-à-dire :
200 µl d'une solution à 15 nM d'un oligonucléotide de séquence 5'-TAAGGCAACATTGCAAGATA-3' couplé à l'extrémité 5' à la phosphatase alcaline, 3 fois 600 µl de solution de lavage PBS Tween et une cuvette de substrat.

Dans le premier puits de la barrette, est déposé 200 µl de la cible à une concentration de 10⁻⁹ M dans le même tampon que pour l'exemple 7.

Après incubation 30 minutes du cône par le mélange cible plus sonde de détection, le cône est lavé 3 fois par une solution de PBS Tween. 250 µl de substrat MUP (méthyl-4 umbelliféryl phosphate) en solution dans un tampon diéthanolamine sont aspirés dans le cône, puis relâchés dans une cuvette de lecture. L'appareil mesure le signal fluorescent exprimé en RFU (unités relatives de fluorescence) de la cuvette.

Les résultats sont représentés à la figure 8 et dans le tableau 11 ci-dessous dans lequel X représente le ligand, *n signifie addition n fois du ligand sur lui-même, b représente l'oligonucléotide de capture tel que représenté précédemment.

**TABLEAU 11**

| X | 1 | 1*2 | 1*3 | 3 | 5 | 6 | 8 | G28K | K20R |
|---|---|---|---|---|---|---|---|---|---|
| Ratio b-X/b | 2,3 | 2,8 | 4,8 | 2,7 | 20,4 | 26,9 | 21,6 | >43 | >43 |

Les résultats confirment l'effet du ligand dans l'amélioration de la sensibilité de détection et le rôle prépondérant des ligands portant au moins un groupement fonctionnel amine primaire.

### EXEMPLE 15

On effectue la détection d'une séquence d'acide nucléique selon le protocole sandwich tel que décrit dans l'exemple 7 en utilisant des plaques de polystyrène irradiées ou non irradiées commercialisées par la société NUNC respectivement sous les références 439454 et 269620.

Le fragment d'acide cible est un fragment d'ADN du papillomavirus de type 16 (région E7) utilisé à une concentration de 10⁻¹⁰ M.

La séquence de l'oligonucléotide de capture utilisé, référencé d, est :

5'-GACAACTGATCTCTAC-3' .

La séquence de l'oligonucléotide de détection, couplé à l'extrémité 5' à la phosphatase alcaline, est :

5′-CCGGACAGAGCCCATTAC-3′

Les résultats sont présentés dans les tableaux ci dessous 12 et 13.

**TABLEAU 12**

| OLIGO -LIGAND | PLAQUE NON IRRADIEE | PLAQUE IRRADIEE |
|---|---|---|
| d | 0,100 D.O. | 0,230 D.O. |
| d-5 | 0,180 D.O. | 0,490 D.O. |

**TABLEAU 13**

| OLIGO-LIGAND | RAPPORT SIGNAL IRRADIEE/NON IRRADIEE |
|---|---|
| d | 2,3 |
| d-5 | 2,7 |

Les résultats ci-dessus confirment le rôle prépondérant du ligand dans la sensibilité de détection. Par ailleurs, on constate une amélioration de la sensibilité de détection lorsqu'on utilise des plaques de microtitration irradiées.

### EXEMPLE 16

On effectue la détection d'une séquence d'acide nucléique selon le protocole sandwich sur l'automate VIDAS selon le protocole décrit dans l'exemple 14 en utilisant des cônes de K résine respectivement irradiés ou non irradiés. Ces cônes sont commercialisées par la société VITEK SYSTEM.

L'ADN, les oligonucléotides de capture et de détection utilisés sont identiques à ceux décrits dans l'exemple 15. Le ligand utilisé est celui référencé précédemment dans l'exemple 5.

Les résultats sont présentés dans le tableau 14 ci-dessous.

**TABLEAU 14.**

| OLIGO -LIGAND | SPR NON IRRADIE | SPR IRRADIE |
|---|---|---|
| d | 84 RFU | 540 RFU |
| d-5 | 1302 RFU | 3917 RFU |

Les rapports de détection SPR activé/SPR non activé sont de :

| OLIGO-LIGAND | RAPPORT activé/non activé |
|---|---|
| d | 6,43 |
| d-5 | >> 3,00 |

Les résultats ci-dessus confirment encore l'effet du ligand sur la sensibilité de détection. Par ailleurs, on constate une diminution du gain du signal lorsqu'on utilise des cônes irradiés, mais ceci est dû à une saturation du détecteur. Le rapport est donc nettement supérieur à 3,00 (>>3,00).

### EXEMPLE 17

On met ci-après en évidence la stabilité des cônes utilisés à différentes températures.

En effet, dans le cas d'utilisation de cônes pour l'automate VIDAS, comme composants de la trousse de détection de fragments d'acides nucléiques, ceux ci doivent être vendus prêts à l'emploi, c'est à dire avec l'oligonucléotide de capture immobilisé. Un facteur déterminant de leur utilisation est leur stabilité dans le temps. Des expériences de stabilité ont été effectuées à trois températures différentes, respectivement 4°, 20°C et 37°C. Les résultats obtenus sont résumés dans le tableau 15 ci-dessous après 42 jours pour chaque température :

**TABLEAU 15**

| TEMPERATURE | RAPPORT SIGNAL jour 42/jour 1 |
|---|---|
| 4° | 100 % |
| 20° | 98 % |
| 37° | 86 % |

Les résultats ci-dessus montrent l'excellente stabilité des cônes utilisés après 42 jours de conservation dans un réfrigérateur à une température de 4°C. Par ailleurs, une augmentation de la température n'affecte que très peu la stabilité des produits.

Comme cela ressort de tous les exemples précédemment décrits, l'addition d'un ligand à un fragment d'acide nucléique augmente dans tous les cas la sensibilité de détection. Cet effet est encore accru lorsqu'on utilise un ligand portant au moins un groupement fonctionnel polaire tel qu'un groupement aminé. Par ailleurs, la fixation du ligand sur le support solide est très facile puisqu'elle se fait directement par adsorption passive. L'hypothèse émise pour expliquer l'adsorption passive directe du ligand sur le support est la combinaison d'interactions hydrophobes entre la partie hydrophobe du ligand et le support et d'interactions polaires entre les groupements polaires du ligand et des groupements polaires du support, ces derniers pouvant être apportés lors du processus de polymérisation du support ou par une irradiation du support après polymérisation.

## Revendications

1. Procédé d'immobilisation par fixation passive non spécifique, sur un support solide, d'un fragment d'acide nucléique comportant moins de 100 nucléotides, dans lequel on dépose sur ledit support ledit fragment sous la forme d'un dérivé résultant du couplage covalent dudit fragment avec un ligand ayant une masse moléculaire inférieure à 5000 et comprenant au moins un groupement fonctionnel polaire, ledit dérivé n'étant pas susceptible de réagir sur ledit support avec formation d'une liaison covalente dans les conditions dudit dépôt, étant entendu que lorsque ledit ligand est un nucléotide ou oligonucléotide, il comprend au moins un nucléotide qui a été modifié de façon à porter ledit groupement fonctionnel polaire.

2. Procédé selon la revendication 1, caractérisé par le fait que ledit groupement fonctionnel est choisi parmi les groupements amine, hydrazino, imino, azido, thiophosphate, hydrazono, sulfamoyle, thiocarboxy, amide éventuellement substitué, triazano, triazéno, amido, semicarbazono, carbamoyle, cyano, carboxyle, hydroxy, alkylthio et arylthio.

3. Procédé selon la revendication 2, caractérisé par le fait que ledit groupement fonctionnel polaire est choisi parmi les groupements amine, hydroxy, amido, carbamoyl, alkylthio, arylthio et thiophosphate.

4. Procédé selon la revendication 3, caractérisé par le fait que ledit groupement amine est choisi parmi les groupements amino, amidino, guanidino, triazinyle, indolyle et imidazolyle.

5. Procédé selon l'une quelconque des revendications précédentes, caractérisé par le fait que ledit ligand comprend au moins un groupement hydrophobe.

6. Procédé selon la revendication 5, caractérisé par le fait que ledit groupement hydrophobe est choisi parmi les groupements hydrocarbonés éventuellement insaturés ayant 2 à 30 atomes de carbone.

7. Procédé selon la revendication 6, caractérisé par le fait que ledit groupement hydrocarboné est choisi parmi les groupements alkyle, alkylène, alcényle, alcénylène, les groupements aryles et arylènes, et les groupements alkyle, alkylène, alcényle ou alcénylène éventuellement interrompus ou substitués par au moins un groupement arylène ou aryle.

8. Procédé selon l'une quelconque des revendications 5 à 7, caractérisé par le fait que ledit ou lesdits groupement(s) hydrophobe(s) est ou sont substitué(s) par ledit ou lesdits groupement(s) fonctionnel(s) polaire(s).

9. Procédé selon l'une quelconque des revendications précédentes, caractérisé par le fait que ledit ligand est lié à un nucléotide terminal dudit fragment.

10. Procédé selon la revendication 9, caractérisé par le fait que ledit ligand est lié à l'extrémité 3' ou 5' dudit fragment.

11. Procédé selon la revendication 9, caractérisé par le fait que ledit ligand est lié à la base dudit nucléotide terminal.

12. Procédé selon l'une quelconque des revendications précédentes, caractérisé par le fait que ledit ligand comprend un groupement choisi parmi :
- les groupements de formule (I) : 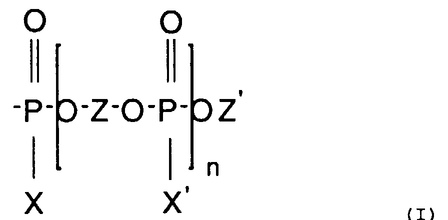 dans laquelle n est zéro ou un nombre entier de 1 à 30,
les groupements Z sont choisis parmi les groupements alkylène et alcénylène ayant 2 à 30 atomes de carbone, Z' est un groupement alkyle ou alcényle ayant 2 à 30 atomes de carbone, X et X' représentent indépendamment -O⁻M⁺, -S⁻M⁺ ou un groupement alkyle, alcoxy, aryloxy, aminoalkyle ou aminoalcoxy, M⁺ étant un cation alcalin ou NH₄⁺, étant entendu que l'un au moins des groupements Z et/ou Z' est substitué par un groupement fonctionnel polaire ou par un groupement portant un groupement fonctionnel polaire ;
- les groupements de formule (II) : 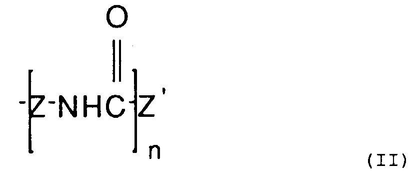 dans laquelle n, Z et Z' sont définis comme précédemment,
- et les groupements de formule (III) :
-L (III)
dans laquelle L est un reste peptidyle comprenant au moins un motif dérivé d'un acide aminé comportant une chaîne latérale substituée par un groupement fonctionnel polaire.

13. Procédé selon la revendication 12, caractérisé par le fait que ledit ligand répond à la formule (I), n étant zéro ou un nombre entier de 1 à 10, Z est un alkylène ayant de 2 à 12 atomes de carbone, et Z' est un alkyle ayant de 2 à 12 atomes de carbone, l'un au moins des groupements Z et Z' étant substitué par au moins un groupement amine, hydroxy ou hydrazino.

14. Procédé selon la revendication 13, caractérisé par le fait que n = O et X représente O⁻M⁺.

15. Procédé selon la revendication 13, caractérisé par le fait que ledit ligand répond à la formule (II), avec n = O, et Z' représente un groupement alkyle ayant 3 à 12 atomes de carbone, substitué par au moins un groupement amine.

16. Procédé selon la revendication 15, caractérisé par le fait que ledit groupement alkyle est en outre substitué par au moins un groupement hydroxy.

17. Procédé selon la revendication 12, caractérisé par le fait que ledit ligand répond à la formule (II), avec n = 1, et Z représente un alkylène ou alcénylène ayant au moins 2 atomes de carbone et Z' représente un groupement alkyle substitué par au moins un groupement amine.

18. Procédé selon la revendication 12, caractérisé par le fait que ledit ligand répond à la formule (II), avec n = 0.

19. Procédé selon la revendication 12, caractérisé par le fait que ledit ligand répond à la formule (III), et que ledit acide aminé comportant une chaîne latérale substituée est choisi parmi la lysine, l'arginine, l'ornithine, la glutamine, l'aspargine, la sérine, la thréonine, la tyrosine, l'histidine et le tryptophane.

20. Procédé selon la revendication 12, caractérisé par le fait que -L est choisi parmi :
- (KGS)ₘ-KGG,m étant un nombre entier pouvant varier de 1 à 15
- KIEPLGVAPTKAKRRWQREKR

21. Procédé selon l'une quelconque des revendications précédentes, caractérisé par le fait que ledit support solide est réalisé en un matériau choisi parmi des polysaccharides comme la cellulose et les dérivés de cellulose tels que l'acétate de cellulose et la nitrocellulose, des polymères vinyliques tels que le polychlorure de vinyle, le polyéthylène, le polystyrène, les dérivés polyacryliques et les polyamides ainsi que des copolymères correspondants.

22. Procédé selon la revendication précédente, caractérisé par le fait que ledit copolymère est un copolymère chlorure de vinyle/acétate de vinyle, chlorure de vinyle/propylène, ou un copolymère de styrène tel qu'un copolymère butadiène-styrène.

23. Procédé selon la revendication précédente, caractérisé par le fait que ledit copolymère styrène contient de 10 à 90 % de poids en motifs styrène.

24. Procédé selon l'une quelconque des revendications 21 à 23, caractérisé par le fait que ledit support est un support modifié par irradiation.

25. Procédé selon l'une quelconque des revendications précédentes, caractérisé par le fait que ledit fragment immobilisé contient moins de 50 et en particulier moins de 30 nucléotides.

26. Support sur lequel est immobilisée par fixation passive une séquence nucléotidique, caractérisé par le fait que ledit support peut être obtenu selon le procédé de l'une quelconque des revendications précédentes.

27. Utilisation d'un support selon la revendication précédente, dans un procédé de détection ou de purification, selon les techniques connues, d'une cible nucléotidique contenant une séquence complémentaire de celle dudit fragment immobilisé.

28. Utilisation selon la revendication précédente, caractérisé par le fait que ledit fragment immobilisé est employé comme sonde de capture.

## Claims

1. Process for immobilization by non-specific passive fixation, on a solid support, of a nucleic acid fragment containing less than 100 nucleotides, in which process the said fragment is deposited on the said support in the form of a derivative resulting from the covalent coupling of the said fragment with a ligand having a molecular mass of less than 5000 and containing at least one polar functional group, the said derivative not being capable of reacting with the said support with the formation of a covalent bond under the conditions of the said deposition, it being understood that, when the said ligand is a nucleotide or oligonucleotide, it comprises at least one nucleotide which has been modified so as to carry the said polar functional group.

2. Process according to Claim 1, characterized in that the said functional group is chosen from amine, hydrazino, imino, azido, thiophosphate, hydrazono, sulphamoyl, thiocarboxyl, optionally substituted amide, triazano, triazeno, amido, semicarbazono, carbamoyl, cyano, carboxyl, hydroxyl, alkylthio and arylthio groups.

3. Process according to Claim 2, characterized in that the said polar functional group is chosen from amine, hydroxyl, amido, carbamoyl, alkylthio, arylthio and thiophosphate groups.

4. Process according to Claim 3, characterized in that the said amine group is chosen from amine, amidino, quanidino, triazinyl, indolyl and imidazolyl groups.

5. Process according to any one of the preceding claims, characterized in that the said ligand contains at least one hydrophobic group.

6. process according to Claim 5, characterized in that the said hydrophobic group is chosen from optionally unsaturated hydrocarbon groups having 2 to 30 carbon atoms.

7. Process according to Claim 6, characterized in that the said hydrocarbon group is chosen from alkyl, alkylene, alkenyl and alkenylene groups, aryl and arylene groups and alkyl, alkylene, alkenyl or alkenylene groups which are optionally interrupted or substituted by at least one arylene or aryl group.

8. Process according to any one of Claims 5 to 7, characterized in that the said hydrophobic group or groups is or are substituted by the said polar functional group or groups.

9. Process according to any one of the preceding claims, characterized in that the said ligand is bonded to a terminal nucleotide of the said fragment.

10. Process according to Claim 9, characterized in that the said ligand is bonded to the 3' or 5' end of the said fragment.

11. Process according to Claim 9, characterized in that the said ligand is bonded to the base of the said terminal nucleotide.

12. Process according to any one of the preceding claims, characterized in that the said ligand contains a group chosen from:
- the groups of formula (I): 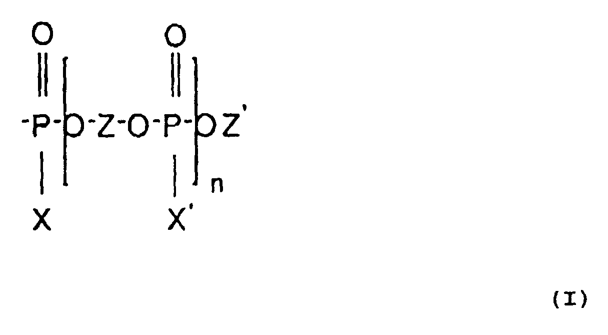 in which n is zero or an integer from 1 to 30,
the groups Z are chosen from alkylene or alkenylene groups having 2 to 30 carbon atoms, Z' is an alkyl or alkenyl group having 2 to 30 carbon atoms and X and X' independently represent -O⁻M⁺, -S⁻M⁺ or an alkyl, alkoxy, aryloxy, aminoalkyl or aminoalkoxy group, M⁺ being an alkali metal cation or NH₄⁺, it being understood that at least one of the groups Z and/or Z' is substituted by a polar functional group or by a group carrying a polar functional group;
- the groups of formula (II): 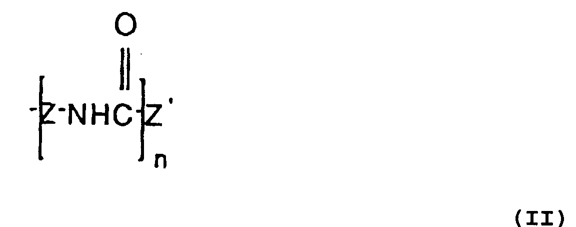 in which n, Z and Z' are defined as above,
- and the groups of formula (III):
-L (III)
in which L is a peptidyl radical comprising at least one unit derived from an amino acid containing a side chain substituted by a polar functional group.

13. Process according to Claim 12, characterized in that the said ligand corresponds to the formula (I), n being zero or an integer from 1 to 10, Z is an alkylene having from 2 to 12 carbon atoms and Z' is an alkyl having from 2 to 12 carbon atoms, at least one of the groups Z and Z' being substituted by at least one amine, hydroxyl or hydrazino group.

14. Process according to Claim 13, characterized in that n = 0 and X represents O⁻M⁺.

15. Process according to Claim 13, characterized in that the said ligand corresponds to the formula (II) where n = 0 and Z' represents an alkyl group having 3 to 12 carbon atoms, substituted by at least one amine group.

16. Process according to Claim 15, characterized in that the said alkyl group is also substituted by at least one hydroxyl group.

17. Process according to Claim 12, characterized in that the said ligand corresponds to the formula (II) where n = 1, Z represents an alkylene or alkenylene having at least 2 carbon atoms and Z' represents an alkyl group substituted by at least one amine group.

18. Process according to Claim 12, characterized in that the said ligand corresponds to the formula (II), where n = 0.

19. Process according to Claim 12, characterized in that the said ligand corresponds to the formula (III) and in that the said amino acid containing a substituted side chain is chosen from lysine, arginine, ornithine, glutamine, asparagine, serine, threonine, tyrosine, histidine and tryptophan.

20. Process according to Claim 12, characterized in that -L is chosen from:
- (KGS)ₘ-KGG, m being an integer which may vary from 1 to 15, and
- KIEPLGVAPTKAKRRWQREKR

21. Process according to any one of the preceding claims, characterized in that the said solid support is produced from a material chosen from polysaccharides, such as cellulose, and cellulose derivatives, such as cellulose acetate and nitrocellulose, vinyl polymers, such as polyvinyl chloride, polyethylene, polystyrene, polyacrylic derivatives and polyamides as well as the corresponding copolymers.

22. Process according to the preceding claim, characterized in that the said copolymer is a vinyl chloride/vinyl acetate or vinyl chloride/propylene copolymer or a styrene copolymer, such as a butadienestyrene copolymer.

23. Process according to the preceding claim, characterized in that the said styrene copolymer contains from 10 to 90% by weight of styrene units.

24. Process according to any one of Claims 21 to 23, characterized in that the said support is a support modified by irradiation.

25. Process according to any one of the preceding claims, characterized in that the said immobilized fragment contains less than 50 and in particular less than 30 nucleotides.

26. Support on which a nucleotide sequence is immobilized by passive fixation, characterized in that the said support may be obtained according to the process of any one of the preceding claims.

27. Use of a support according to the preceding claim, in a process for the detection or the purification, in accordance with known techniques, of a nucleotide target containing a sequence complementary to that of the said immobilized fragment.

28. Use according to the preceding claim, characterized in that the said immobilized fragment is used as capture probe.

## Patentansprüche

1. Verfahren zur Immobilisierung eines Nukleinsäure-Fragments mit weniger als 100 Nukleotiden durch passive, nicht-spezifische Fixierung auf einem festen Träger, bei dem man auf dem Träger das Fragment in Form eines Derivats aufbringt, das durch kovalente Kupplung des Fragments mit einem Liganden erhalten wird, der ein Molekulargewicht unter 5000 und mindestens eine funktionelle, polare Gruppe aufweist, wobei das Derivat nicht geeignet ist, unter Bildung einer kovalenten Bindung unter den genannten Bedingungen des Aufbringens zu reagieren, der Ligand ein Nukleotid oder Oligonukleotid ist und mindestens ein Nukleotid umfaßt, das auf eine Weise modifiziert wurde, daß es die funktionelle, polare Gruppe trägt.

2. Verfahren gemäß Anspruch 1, dadurch gekennzeichnet, daß die funktionelle Gruppe ausgewählt ist aus den Gruppen: Amin, Hydrazin, Imino, Azido, Thiophosphat, Hydrazon, Sulfamoyl, Thiocarboxy, gegebenenfalls substituiertes Amid, Triazan, Triazen, Amido, Semicarbazon, Carbamoyl, Cyano, Carboxyl, Hydroxy, Alkylthio und Arylthio.

3. Verfahren nach Anspruch 2, dadurch gekennzeichnet, daß die funktionelle, polare Gruppe ausgewählt wird aus den Gruppen: Amin, Hydroxy, Amido, Carbamoyl, Alkylthio, Arylthio und Thiosphosphat.

4. Verfahren nach Anspruch 3, dadurch gekennzeichnet, daß die Amingruppe ausgewählt ist unter den Gruppen: Amino, Amidino, Guanidino, Triazinyl, Indolyl und Imidazolyl.

5. Verfahren nach einem der vorstehenden Ansprüche, dadurch gekennzeichnet, daß der Ligand mindestens eine hydrophobe Gruppe aufweist.

6. Verfahren nach Anspruch 5, dadurch gekennzeichnet, daß die hydrophobe Gruppe unter gegebenenfalls ungesättigten Kohlenwasserstoffgruppen mit 2 bis 30 Kohlenstoffatomen ausgewählt wird.

7. Verfahren nach Anspruch 6, dadurch gekennzeichnet, daß die Kohlenwasserstoffgruppe aus Alkyl-, Alkylen-, Alkenyl-, Alkenylengruppen, Aryl- und Arylengruppen oder Alkyl-, Alkylen-, Alkenyl- oder Alkenylengruppen ausgewählt ist, die gegebenenfalls durch mindestens eine Arylen- oder Arylgruppe unterbrochen oder substituiert sein können.

8. Verfahren nach einem der Ansprüche 5 bis 7, dadurch gekennzeichnet, daß die hydrophobe(n) Gruppe(n) durch die genannten funktionellen, polaren Gruppen substituiert ist bzw. sind.

9. Verfahren nach einem der vorstehenden Ansprüche, dadurch gekennzeichnet, daß der Ligand an ein terminales Nukleotid des Fragments gebunden ist.

10. Verfahren nach Anspruch 9, dadurch gekennzeichnet, daß der Ligand an das 3'- oder 5'-Ende des Fragments gebunden ist.

11. Verfahren nach Anspruch 9, dadurch gekennzeichnet, daß der Ligand an die Base des terminalen Nukleotids gebunden ist.

12. Verfahren nach einem der vorstehenden Ansprüche, dadurch gekenzeichnet, daß der Ligand eine Gruppe umfaßt, ausgewählt aus:
- Gruppen der Formel (1): 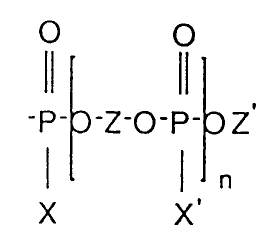 in der n Null oder eine ganze Zahl von 1 bis 30 bedeutet,
die Gruppen Z ausgewählt sind aus Alkylen- und Alkenylengruppen mit 2 bis 30 Kohlenstoffatomen, Z' eine Alkyl- oder Alkenylgruppe mit 2 bis 30 Kohlenstoffatomen bedeutet, X und X' unabhängig -O⁻M⁺, -S⁻M⁺oder eine Alkyl-, Alkoxy-, Aryloxy-, Aminoalkyl- oder Aminoalkoxygruppe bedeuten, M⁺ ein alkalisches Kation oder NH₄⁺ bedeutet, mit der Maßgabe, daß mindestens eine der Gruppen Z und/oder Z' durch eine funktionelle, polare Gruppe oder eine Gruppe substituiert sein kann, die eine funktionelle, polare Gruppe trägt;
- Gruppen der Formel (II): 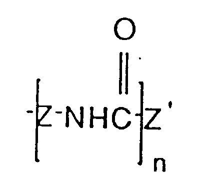 in der n, Z und Z' die oben angegebene Bedeutung aufweisen,
- Gruppen der Formel (III):
-L (III)
in der L einen Peptidylrest bedeutet, der mindestens eine Einheit aufweist, die von einer Aminosäure abgeleitet ist, die eine Seitenkette aufweist, die durch eine funktionelle, polare Gruppe substituiert ist.

13. Verfahren nach Anspruch 12, dadurch gekennzeichnet, daß der Ligand der Formel (I) entspricht, n Null oder eine ganze Zahl von 1 bis 10 bedeutet, Z ein Alkylen mit 2 bis 12 Kohlenstoffatomen ist, Z' ein Alkyl mit 2 bis 12 Kohlenstoffatomen bedeutet, wobei mindestens eine der Gruppen Z und Z' durch mindestens eine Amin-, Hydroxy- oder Hydrazingruppe substituiert sind.

14. Verfahren nach Anspruch 13, dadurch gekennzeichnet, daß n = 0 und X O⁻M⁺ bedeuten.

15. Verfahren nach Anspruch 13, dadurch gekennzeichnet, daß der Ligand der Formel (II) entspricht, wobei n = 0 bedeutet und Z' eine Alkylgruppe mit 3 bis 12 Kohlenstoffatomen ist, die mindestens durch eine Amingruppe substituiert ist.

16. Verfahren nach Anspruch 15, dadurch gekennzeichnet, daß die Alkylgruppe außerdem mindestens durch eine Hydroxygruppe substituiert ist.

17. Verfahren nach Anspruch 12, dadurch gekennzeichnet, daß der Ligand der Formel (II) entspricht, wobei n = 1 bedeutet, Z ein Alkylen oder Alkenylen mit mindestens 2 Kohlenstoffatomen ist und Z' eine mindestens durch eine Amingruppe substituierte Alkylgruppe bedeutet.

18. Verfahren nach Anspruch 12, dadurch gekennzeichnet, daß der Ligand der Formel (II) entspricht, wobei n = 0 ist.

19. Verfahren nach Anspruch 12, dadurch gekennzeichnet, daß der Ligand der Formel (III) entspricht und die eine substituierte Seitenkette tragende Aminosäure ausgewählt ist aus: Lysin, Arginin, Ornithin, Glutamin, Asparagin, Serin, Threonin, Tyrosin, Histidin und Tryptophan.

20. Verfahren nach Anspruch 12, dadurch gekennzeichnet, daß -L ausgewählt ist aus:
- (KGS)ₘ-KGG, wobei m eine ganze Zahl von 1 bis 15 bedeutet,
- KIEPLGVAPTKAKRRWQREKR.

21. Verfahren nach einem der vorstehenden Ansprüche, dadurch gekennzeichnet, daß der feste Träger aus einem Material hergestellt wird, ausgewählt aus Polysacchariden wie Cellulose und Cellulosederivaten wie Celluloseacetat und Nitrocellulose, Vinylpolymeren wie Vinylpolychlorid, Polyethylen, Polystyrol, Polyacylsäurederivaten und Polyamiden sowie den entsprechenden Copolymerisaten.

22. Verfahren nach dem vorstehenden Anspruch, dadurch gekennzeichnet, daß das Copolymere ein Vinylchlorid/Vinylacetat-Copolymeres, Vinylchlorid/Propylen-Copolymeres, oder ein Copolymeres des Styrols wie Butadien/Styrol ist.

23. Verfahren nach dem vorstehenden Anspruch, dadurch gekennzeichnet, daß das Styrol-Copolymerisat 10 bis 90 Gew.-% Styrol-Einheiten enthält.

24. Verfahren nach einem der Ansprüche 21 bis 23, dadurch gekennzeichnet, daß der Träger ein durch Strahlung modifizierter Träger ist.

25. Verfahren nach einem der vorstehenden Ansprüche, dadurch gekennzeichnet, daß das immobilisierte Fragment mindestens 50, im besonderen mindestens 30 Nukleotide enthält.

26. Träger, auf dem durch passive Fixierung eine Nukleotid-Sequenz immobilisiert ist, dadurch gekennzeichnet, daß der Träger nach einem der Verfahren der vorstehenden Ansprüche erhalten wurde.

27. Verwendung eines Trägers nach dem vorstehenden Anspruch, in einem bekannten Identifizierungs- oder Reinigungs-Verfahren für ein Nukleotid-Target, enthaltend eine zu dem immobilisierten Fragment komplementäre Sequenz.

28. Verfahren nach dem vorstehenden Anspruch, dadurch gekennzeichnet, daß das immobilisierte Fragment als Fänger-Sonde verwendet wird.
